# EUROPEAN PATENT SPECIFICATION

(11) **EP 3 788 948 B1**
(45) Date of publication and mention of the grant of the patent: **02.09.2026**
(21) Application number: 19382757.3
(22) Date of filing: 04.09.2019
(51) Int. Cl.: A61B 5/00, A61B 5/145, A61B 5/1486, A61B 5/1455

(54) **IMPLANTABLE ELECTRONIC SENSING SYSTEM FOR MEASURING AND MONITORING MEDICAL PARAMETERS**
IMPLANTIERBARES ELEKTRONISCHES MESSSYSTEM ZUR MESSUNG UND ÜBERWACHUNG VON MEDIZINISCHEN PARAMETERN
SYSTÈME DE DÉTECTION ÉLECTRONIQUE IMPLANTABLE PERMETTANT DE MESURER ET DE SURVEILLER DES PARAMÈTRES MÉDICAUX

(43) Date of publication of application: 10.03.2021
(73) Proprietor: Universitat Pompeu Fabra, 08002 Barcelona (ES)
(72) Inventor: IVORRA CANO, Antoni, 08002 Barcelona (ES); CASTELLVÍ FERNÁNDEZ, Quim, 08002 Barcelona (ES); BECERRA FAJARDO, Laura, 08002 Barcelona (ES)
(74) Representative: TRBL Intellectual Property

(56) References cited:
- US-A1- 2019 150 884
- LAURA BECERRA-FAJARDO ET AL: "Bidirectional communications in wireless microstimulators based on electronic rectification of epidermically applied currents", 2013 6TH INTERNATIONAL IEEE/EMBS CONFERENCE ON NEURAL ENGINEERING (NER), 1 April 2015 (2015-04-01), pages 545 - 548, XP055669310, ISSN: 1948-3546, DOI: 10.1109/NER.2015.7146680
- L BECERRA-FAJARDO ET AL: "Towards addressable wireless microstimulators based on electronic rectification of epidermically applied currents", 2014 36TH ANNUAL INTERNATIONAL CONFERENCE OF THE IEEE ENGINEERING IN MEDICINE AND BIOLOGY SOCIETY, vol. 2014, 1 August 2014 (2014-08-01), United States, pages 3973 - 3976, XP055670541, ISSN: 1094-687X, DOI: 10.1109/EMBC.2014.6944494
- "Replace, Repair, Restore, Relieve - Bridging Clinical and Engineering Solutions in Neurorehabilitation", vol. 7, 1 January 2014, SPRINGER INTERNATIONAL PUBLISHING, Cham, ISBN: 978-3-319-08072-7, ISSN: 2195-3562, article ANTONI IVORRA ET AL: "Flexible Thread-like Electrical Stimulation Implants Based on Rectification of Epidermically Applied Currents Which Perform Charge Balance", pages: 447 - 455, XP055670551, DOI: 10.1007/978-3-319-08072-7_67
- SHUENN-YUH LEE ET AL: "An implantable wireless bidirectional communication microstimulator for neuromuscular stimulation", IEEE TRANSACTIONS ON CIRCUITS AND SYSTEMS PART I: REGULAR PAPERS., vol. 52, no. 12, 1 December 2005 (2005-12-01), US, pages 2526 - 2538, XP055669304, ISSN: 1057-7122, DOI: 10.1109/TCSI.2005.857770
- LAURA BECERRA-FAJARDO ET AL: "Demonstration of 2 mm Thick Microcontrolled Injectable Stimulators Based on Rectification of High Frequency Current Bursts", IEEE TRANSACTIONS ON NEURAL SYSTEMS AND REHABILITATIONENGINEERING., vol. 25, no. 8, 1 August 2017 (2017-08-01), US, pages 1343 - 1352, XP055669120, ISSN: 1534-4320, DOI: 10.1109/TNSRE.2016.2623483
- "IFMBE proceedings (International Federation for Medical and Biological Engineering)", vol. 68/3, 30 May 2018, SPRINGER, DE, ISSN: 1680-0737, article LAURA BECERRA-FAJARDO ET AL: "First Steps Towards an Implantable Electromyography (EMG) Sensor Powered and Controlled by Galvanic Coupling", pages: 19 - 22, XP055669126, DOI: 10.1007/978-981-10-9023-3_4
- "IFMBE proceedings (International Federation for Medical and Biological Engineering)", vol. 68/3, 30 May 2018, SPRINGER, DE, ISSN: 1680-0737, article MARC TUDELA-PI ET AL: "Powering Implants by Galvanic Coupling: A Validated Analytical Model Predicts Powers Above 1 mW in Injectable Implants", pages: 23 - 26, XP055669279, DOI: 10.1007/978-981-10-9023-3_5
- ANTONI IVORRA ET AL: "demonstration of injectable microstimulators based on charge-balanced rectification of epidermically applied currents", JOURNAL OF NEURAL ENGINEERING, INSTITUTE OF PHYSICS PUBLISHING, BRISTOL, GB, vol. 12, no. 6, 8 October 2015 (2015-10-08), pages 66010, XP020293214, ISSN: 1741-2552, [retrieved on 20151008], DOI: 10.1088/1741-2560/12/6/066010

## Description

### Object of the invention

The present invention generally refers to implantable sensing systems for measuring and monitoring medical parameters from a living human or animal body.

More specifically, the invention refers to an electronic implant and to a reading unit to obtain measurements originating at the implant or its surroundings to characterize physical and/or chemical clinical parameters of a living body for medical and biomedical applications.

An object of the invention is to provide an electronic implant of reduced dimensions, that features a minimal invasiveness during its implantation, in such a way that the implant can be implanted by injection or by catheterization rather than by open surgery.

The system of the invention can advantageously be used for example for monitoring: congestive heart failure, blood pressure in vessels, pulse oximetry, blood glucose, tissue ischemia, and tissue edema among others.

### Background of the invention

Implantable sensing systems have been developed to measure and monitor clinically relevant magnitudes in a living body for medical applications such as: blood glucose for diabetic patients, pH for monitoring gastroesophageal reflux, blood pressure for monitoring heart failure, bladder pressure in urinary incontinence patients etc. In contrast to external sensing systems, implantable systems are capable of detecting the stimuli where they originate and that provides them with higher accuracy.

These implantable sensing systems are normally used for diagnosis and for determining treatment dosage and timing. Surprisingly, the number of commercially available products is very reduced compared with the large amount of published academic research in the field of implantable sensors. Such disparity is due to market and regulatory constraints and, more importantly, to practical factors such as the invasiveness of the available implants.

Therefore, implantable sensing systems that are minimally invasive either because the implantation of the devices is done by catherization or by injection, or because the systems re-use an already surgically implanted device, are by far, preferred over systems that require highly invasive surgeries, even if the performance of the non-invasive products is lower than the surgically implanted ones.

A known type of electronic sensing implants are based on active electronics. These implants incorporate a mechanism to generate electric energy to power an electronic circuit capable of reading and processing signals from a sensor, and transmitting the result to an external unit for further processing or representation. In some cases, the electric power is entirely generated internally (e.g. with electrochemical batteries) and in other cases the electric power is either generated by transforming a sort of energy already present in the body (e.g. so-called energy harvesters that can transform kinematic energy into electric energy), or by wireless power transmission from an external unit (e.g. by ultrasound power transmission or by inductive coupling power transmission).

In the above cases, the mechanism for generating the electric energy requires bulky components that hinder miniaturization of the implants, in such a way that implantation by catheterization or injection is not feasible.

Another type of known electronic sensing implants are those based on passive electronics, that are those that do not contain a mechanism to power a circuit. Most of the commercially available systems of this type are based on combinations of inductors and capacitors (LC systems) that resonate at a specific frequency when an alternating magnetic field is applied. Such frequency is typically determined by a capacitor which acts as the sensor, as its capacitance depends on the magnitude of interest. The main disadvantage of these systems is that they require coils with a relatively large diameter, both at the implant and at the external unit, especially if the device is intended for deep implantation.

In non-electronic sensing implants, the magnitude of interest is transduced into a non-electrical magnitude which is read and processed by a reading unit. Some available systems of this type are based on transduction into optic properties and, in particular, into fluorescence. These systems exhibit some significant drawbacks, for instance, since these systems use chemical reactions, the operating life of the implants is short. In addition, these systems are limited to applications in which the sensor is very close to the reading unit, so that optical transmission is feasible.

Implantable sensing systems may be of use for blood pressure monitoring. Long term and continuous blood pressure monitoring is required for a number of clinical needs, which are unmet by conventional blood pressure measurement systems based on sphygmomanometers or catheter pressure sensors. These two technologies are too obtrusive for long term and continuous blood pressure monitoring. Attempts have been carried out to implement implantable pressure sensors to monitor blood pressure in a non-obtrusive way. However, until now the developed implantable systems are relatively bulky and are not adequate for deployment in the narrow arteries and veins of the peripheral vascular system which would be preferable over implantation in vessels of the abdomen or the thorax for minimizing risks.

Another case in which implantable sensing systems may be of use is for detecting congestive heart failure based on impedance measurements of the lungs. Existing implantable pacemakers and defibrillators incorporate a similar functionality: they provide measurements of the so-called transthoracic impedance for early detection of worsening heart failure. Those measurements are obtained by performing impedance measurements across the electrodes they possess for their therapeutic function. However, since these electrodes are implanted within large blood cavities (i.e. the heart ventricles and auriculas) and the conductivity of the blood is much higher than that of lung tissues, the measurements that these systems perform exhibit low sensitivity to the conductivity of the lungs and, consequently, they exhibit a large rate of false positives. Furthermore, it must be noticed that only a minority of patients with congestive heart failure are implanted with a defibrillator or a pacemaker.

In the scientific publications (Conf. Proc. ICNR2014 447-455 doi: 10.1007/978-3-319-08072-7_67, J Neural Eng. 2015 12(6):066010 doi: 10.1088/1741-2560/12/6/066010) and the patent US 9,446,255 the use of implants for stimulation is disclosed. However, these publications only relate to stimulation, they are silent to the use of these implants for measurement or monitoring parameters in a living body.

U.S. patents: US 8,725,270 and US 8,909,343 describe the use of implants based on a single diode for sensing the impedance of tissues and biopotentials, by processing the harmonics generated by the diode when a radiofrequency electromagnetic wave is applied. In these patents, since the diode is the only sensing element, there are constrains with the magnitudes that can be sensed, and the accuracy of the measurements is reduced.

It is known to use the volume conduction property of human tissue as a natural medium for delivery of energy. For example, the PCT publication WO2006105245 (A2) discloses the use of the volume conduction for energy delivery to implants. The disclosure of this PCT publication is focused on an external antenna design, that consists of an array of

electrodes arranged to receive voltage and work collaboratively to transmit electrical energy to a target site, wherein the external delivery of electrical energy from outside the human body to a target site within the human body is carried out, such as electrical stimulation of muscles and power delivery to implanted devices.

This PCT publication is silent to the use of electronic rectification of volume conducted currents to obtain measurements originating at the implant or its surroundings to characterize physical and/or chemical clinical parameters of a living body for medical and biomedical applications. It is also silent about the structure of the implants.

Therefore, there is the need in this technical field to further reduce the dimensions and invasiveness of implantable sensing systems, in such a way that these systems can be easily deployed by injection or by catheterization.

Publication: LAURA BECERRA-FAJARDO ET AL, "Bidirectional communications in wireless microstimulators based on electronic rectification of epidermically applied currents", 2013 6TH INTERNATIONAL IEEE/EMBS CONFERENCE ON NEURAL ENGINEERING (NER), (20150401), doi:10.1109/NER.2015.7146680, ISSN 1948-3546, pages 545 - 548, XP055669310, relates to techniques for electrical stimulation to restore muscle functions of patients suffering neurological disorders.

### Summary of the invention

The invention is defined in the attached independent claim 1, including the preferred embodiments defined in the dependent claims. The invention satisfactorily solves the drawbacks of the prior art, by providing an implant that exhibits reduced invasiveness, with a thickness ranging from few millimeters to fractions of a millimeter (e.g. 0.5 mm), such as the implant can be deployed by injection or by catheterization.

The invention refers to a sensing system comprising at least one implant and a reading unit cooperating with the implant to obtain accurate measurements to characterize physical and/or chemical clinical parameters of a living body.

An aspect of the invention refers to an implant as defined by claim 1 comprising an electronic circuit and at least two electrodes connected to the electronic circuit, wherein the circuit comprises a capacitor and a device of asymmetric conductance, both connected in series between two electrodes. The device of asymmetric conductance is a two-terminal electronic component or system capable of controlling the direction of current flow through its two terminals, for example a diode, a p-n junction of a transistor or a smart diode.

The capacitor prevents that dc currents flow through the implant, as these dc currents would cause irreversible electrochemical reactions at the implant electrodes, that in turn would damage both the electrodes and the living tissues.

The implant circuit additionally comprises a discharge network connected in parallel with the device of asymmetric conductance for the capacitor discharge, wherein the discharge network comprises at least one electrical or electronic component, for example a resistor and/or a semiconductor device, so that the capacitor discharges through the discharge network and through a medium of a living body in which the implant is immersed.

According to the invention, the capacitor, the device of asymmetric conductance and/or the electrical or electronic component of the discharge network, are a transducer selected so that one of its operational parameters is variable depending on a physical and/or chemical condition of a medium in which the implant might be implanted for measuring a medical parameter. The medium consists of a tissue or a fluid (e.g. blood) of a human or animal body.

For example, the capacitor or the resistor of the discharge network, can be implemented as a transducer whose characteristic value (capacitance (C) and resistance (R), respectively) depends on a physical or chemical magnitude of interest (i.e. measurand), and a reading unit can derive the value of those magnitudes (i.e. measurements) by processing the characterization. It is well known that if the function that relates the measurand (*x*) and the transducer output is established (*y = f*(*x*)), it is possible to compute a measurement by applying the inverse function (*x*' = *f*⁻¹(*y*)).

The separation distance between the implant electrodes should be large enough so as to pick up a voltage difference sufficient for the operation of the implant circuit. That is, the separation distance must be larger than the minimum voltage for operation, divided by the expected electric field magnitude at the location of the implant.

Preferably, the device of asymmetric conductance is a diode that requires a minimum voltage in the order of hundreds of millivolts for operation. Taking into account that the maximum allowable electric field amplitude at the location of the implant will be in the order of a few volts per centimeter, the diode voltage requirement translates in a minimum inter electrode distance in the order of a few millimeters. Considering that, when implanted, the implant might not be perfectly aligned with the electric field and other uncertainties, the separation distance between the electrodes, and hence the length of the implant, will be in the order of a centimeter or a very few centimeters, preferably the length of the implant is within the range 0.5 - 5 cm.

In a preferred embodiment, the discharge network consists in a resistor of a given nominal value, and the capacitor is a transducer whose capacitance is variable depending on a physical and/or chemical condition of the medium. Preferably, the capacitor is a pressure capacitor whose capacitance depends on the pressure applied to a part of the capacitor.

In another preferred embodiment, the capacitor has a given nominal capacitance, and the electrical or electronic component of the discharge network, is a resistive transducer, preferably a thermistor or a light dependent resistor (LDR).

In another preferred embodiment, the discharge network contains a current controlling device, such as a current-limiting diode or a JFET current limiter, that controls the discharge current and makes it independent of the impedance of the medium. Such current controlling device may be of nominal current or may be configured to depend on a measurand. For instance, the resistor in a JFET current limiter may be a resistive transducer.

In another preferred embodiment, the implant is adapted for sensing biopotentials or chemical species. In this case, the capacitor has a given nominal capacitance and the electronic component of the discharge network is a transistor configured as a transducer in parallel with the device of asymmetric conductance. The gate or base terminal of the transistor, is in contact with the medium in such a way that the conductance of the transistor depends on voltage gradients at the medium or on the concentration of an ion in the medium. For sensing ion concentrations, the gate or base terminal of the transistor is in contact with the medium directly or through a so-called ion selective membrane. For sensing voltage gradients, the gate or base terminal of the transistor is in contact with the medium through a third electrode.

In this embodiment, the discharge network may additionally comprise an auxiliary diode connected in series with the transistor. The anode of the auxiliary diode is connected to the cathode of the device of asymmetric conductance and to one terminal of the capacitor, and the cathode of the auxiliary diode is connected to the collector or drain of the transistor. The source or emitter of the transistor is connected to the anode of the device of asymmetric conductance, and to one electrode of the implant.

In another preferred embodiment, the device of asymmetric conductance and the electrical or electronic component of the discharge network, cooperate to configure an optical transducer together with an optical reactive material, in such a way that an optical property of the optical transducer is variable depending on a physical or chemical condition of the medium. The optical reactive material, preferably a fluorescence or a phosphorescence variable material, is arranged in the implant to be in contact with the medium when the implant is in use.

In this embodiment, the device of asymmetric conductance is a light emitting semiconductor device that emits light when a current passes through it, and the electronic component of the discharging network is a light sensitive (receiving) conductive device, that allows current flow when it receives a suitable intensity of light. The optical reactive material is arranged to transmit light from the light emitting semiconductor device or to generate light after being illuminated by the light emitting semiconductor device, to the light sensitive conductive device, so that the capacitor can discharge through the light sensitive conductive device, when this device is activated by the light received from the optical reactive material.

The light emitting semiconductor device and the light sensitive (receiving) conductive device, can be implemented as optoelectronic components, for example as a photoemitter and a photodetector (photodiode) respectively. In order to increase the selectivity of this type of implants based on optoelectronic components and, in particular, when the fluorescence of the transducer material is characterized, preferably optical filters or diffraction grids are placed on the photoemitter and on the photodetector (photodiode) in order to select specific light wavelengths or bands of operation.

In another preferred embodiment, the device of asymmetric conductance is a diode, the capacitor has a fixed nominal value, and the implant further comprises a transmitting, reflecting, or refractive optical reactive material. The discharge network comprises a light emitting semiconductor device and a light sensitive conductive device, both connected in parallel with the device of asymmetric conductance. The optical material is arranged to transmit light from the light emitting semiconductor device to the light sensitive conductive device, so that the capacitor can be discharged through the light sensitive conductive device to emit light during the capacitor discharge.

Preferably, for the above optical embodiments, the implant includes a capsule where the electronic implant circuit is housed, and at least a part of the capsule is formed with the optical reactive material described above.

In another preferred embodiment, the optical reactive material is a deformable material whose optical transmissivity varies depending on the pressure or force applied to it, thus, the implant circuit operation depends on the pressure or force applied externally to the capsule.

In another preferred embodiment, the implant is suitable to be deployed inside an artery or a vein for measuring blood pressure. In this case, the implant comprises a capsule having at least a part made of a flexible material that allows pressure transmission from the exterior to the capsule interior. The implant circuit is hermetically housed within the capsule, and the two electrodes of the implant pass through the capsule and are connected to the implant circuit. Preferably, each electrode is a flexible wire conformed as a loop.

In another preferred embodiment, the capacitor and the flexible part of the capsule, are arranged relative to each other such as the capacitance varies with the deformation of the flexible material.

Another aspect of the invention refers to an implantable sensing system comprising at least one electronic implant as any one of the alternative implant configurations described above, and a reading unit for interrogating the electronic implant or implants deployed in a living body. The reading unit comprises: two or more electrodes like surface or skin electrodes, an alternating voltage generator to generate an alternating voltage across the electrodes, and a control and processing module for controlling the voltage generator. The reading unit is configured for emitting bursts of an alternating current, preferably high frequency currents, suitable to reach an implant deployed internally inside a body by volume conduction (galvanic conduction) through the body.

The reading unit is additionally adapted to interrogate an implant by delivering bursts of high frequency electric currents through electrodes. The measurement is obtained by processing the voltage or current signals that result during or after the delivery of the bursts. The voltage signals are measured from the same two electrodes that deliver the current of electric currents, or alternatively from the electrodes in contact with the tissues where the implant is deployed.

Preferably, the implant is a thread-shaped and flexible body with two electrodes at opposite ends, to allow minimally invasive percutaneous deployment.

The invention provides alternative implant circuit architectures, adapted for capturing different measurements. These circuit architectures are implemented either by using discrete components mounted on a micro-circuit board, or by integrating them monolithically in an integrated circuit or microsystem using semiconductor manufacturing technology.

The reading or interrogation method between the reading unit and an implant, comprises the following stages:
1.- applying bursts of alternating current, for example sinusoidal, generated by the reading unit that reach an implant by volume conduction,
2.- detecting resulting voltage and/or current signals at the reading unit,
3.- processing those signals to characterize the capacitance of the implant capacitor, the resistance of the discharging element, or the impedance of the tissues that surround the implant and
4.- processing the characterization for obtaining a measurement of interest.

Preferably, the reading unit is a battery powered hand-help unit, embodied as an external device. Alternatively, the reading unit is formed by two parts, namely: an implantable part (e.g. battery or inductively powered) for reading an implant inside a body, and an external part wirelessly communicated with the implantable part. In particular, the implantable part can consist of a subcutaneously implanted sub-unit capable of generating current bursts and also for taking measurements. In turn, the external sub-unit is capable of processing data wirelessly transmitted by the implanted sub-unit, and for representing the measurements for example in a display and for generating alarms.

Multiple implants can be independently interrogated if they are sufficiently separated within the body. The separation must be enough to ensure that only the implant of interest is powered or activated when the bursts are delivered. That is, in the case that the implants are not intended to be interrogated, it should be ensured that the electric field that reaches them is low enough to prevent conduction of the device of asymmetric conductance (e.g. diode).

Alternatively, multiple implants can be simultaneously energized for operation and selectivity can be achieved by the relative location of the voltage pick-up electrodes of the reading unit.

For all the embodiments of the invention, the injected alternating currents for interrogation, which are either current controlled or voltage controlled, are specially selected to be innocuous to the body. This is accomplished by ensuring that these currents are of sufficient frequency to prevent unsought stimulation of excitable tissues (first requirement), and their power is low enough to prevent excessive heating of tissues due to Joule heating (second requirement).

The first requirement can be readily met by using currents whose power spectral density is well above 100 kHz. For instance, sinusoidal currents with a frequency (f) of or above 1 MHz, are desired. Furthermore, frequencies below 100 MHz are preferred, to prevent that the skin effect becomes significant, and the operation of implants at deep locations is hindered.

The second requirement is achieved by delivering short bursts. For a given voltage gradient (*E*) required to ensure operation of the implants (e.g. 200 V/m), burst duration (*B*) and burst repetition frequency (*F*), are selected to ensure that power dissipation in the tissue where the implants are located does not reach a safety threshold.

Safety thresholds for electromagnetic power dissipation in tissues are usually specified by the so-called Specific Absorption Rate (SAR), which is measured in W/kg. SAR can be calculated with the following expression: $SAR = \frac{σ {\left({E}_{RMS}\right)}^{2}}{ρ}$ where *σ* is the electrical conductivity of the tissue (S/m), *ρ* is the mass density of the tissue (kg/m³) and *E_{RMS}* is the root mean square value of the electric field in the tissue (V/m). From that expression it can be obtained the following requirement for *F* and *B* in the case of sinusoidal bursts: $FB < \frac{2 ρ {SAR}_{MAX}}{{σE}^{2}}$

A SAR value of 2 W/kg is considered to be a safe threshold in all circumstances according to different standards. If the above expression is particularized for the case of muscle tissue at 10 MHz (*σ* _{10 MHz} = 0.62 S/m, *ρ* = 1060 kg/m³) and it is assumed that the field required for operation is 200 V/m, the 2 W/kg limit yields *FB <* 0.17 s/s. Thus, for instance, if the burst duration, is 10 µs, the maximum repetition frequency would be 17 kHz.

It must be noted that the SAR limitation must not only be met where the implants are located but also in all tissue regions where the interrogation currents flow through. Therefore, since current densities (and voltage gradients) will be probably higher in the vicinity of the current injecting electrodes of the reading unit, the *FB* product will have to be scaled down.

Preferably, the interrogation signal consists of a sinusoidal waveform with a frequency between 100 kHz and 100 MHz which is delivered as bursts with a duration between 0.1 µs and 10 ms, and a repetition frequency between 0 Hz (i.e. single burst interrogation) and 100 kHz.

Some of the advantages of the system of the invention are summarized below:
- minimal invasiveness during its implantation, as the implant can be deployed by injection or by catheterization,
- implantation within body locations that were not feasible before,
- accurate measurements compared with external measurement devices,
- low cost implants and simplification of external reading unit.

### Brief description of the drawings

Preferred embodiments of the invention, are henceforth described with reference to the accompanying drawings, wherein:
Figure 1.- shows a schematic representation of the implantable sensing system of the invention, wherein an implant is shown deployed inside a body while it is being interrogated by an external reading unit.
Figure 2.- shows an electric diagram of a preferred circuit architecture for an implant according to the invention.
Figure 3.- shows an electric diagram of the preferred circuit architecture of figure 2, together with the impedance of the electrodes, and the Thévenin equivalent circuit that the implant perceives.
Figure 4.- shows an electric diagram of another preferred embodiment of an implant circuit according to the invention, for performing measurements with a capacitive transducer.
Figure 5.- shows an electric diagram of another preferred embodiment of an implant circuit according to the invention, for performing measurements with a resistive transducer instead of a capacitance transducer.
Figure 6.- shows an electric diagram of another preferred embodiment of an implant circuit according to the invention, for performing voltage measurements.
Figure 7.- shows an electric diagram of another preferred embodiment of an implant circuit according to the invention, for performing ion concentration measurements based on an ISFET. Ionic conductance is indicated by means of a dotted line.
Figure 8.- shows an electric diagram of another preferred embodiment of an implant circuit according to the invention, for performing measurements of the phosphorescence of a transducer material. Light transmission is indicated with arrows.
Figure 9.- shows a schematic representation of a cross-sectional view of another preferred embodiment of the invention, that comprises a capsule material capable of transducing a magnitude of interest (e.g. oxygen concentration) into an optical property (e.g. phosphorescence). Light transmission through the capsule material is represented by means of a broken line.
Figure 10.- shows an electric diagram of another preferred embodiment of an implant circuit according to the invention, for performing measurements of materials that do not exhibit phosphorescence.
Figure 11.- shows an electric diagram of the circuit of Figure 2 with impedance models for the implant and the reading unit electrodes, and an impedance two-port model for the living tissues that couples the reading unit to the implant.
Figure 12.- shows a graph illustrating the fitting to an exponential decay function by using least squares fitting. Fitting is performed on the average of 100 recordings of the sensed voltage.
Figure 13.- shows a schematic representation of an implantable sensing system according to the invention, including the electric diagram of the reading unit that allows recording the sensed voltage with the same pair of electrodes used for delivering the interrogation signals.
Figure 14.- shows an electric diagram of a simplified implant circuit architecture together with the resistances model for the living tissues and the generator of the reading unit.
Figure 15.- shows examples of unbalanced current amplitude between semi-cycles at different voltage amplitudes applied by the reading unit at: (a) As measured at the reading unit, and (b) as measured at the implant.
Figure 16.- shows a schematic representation of an embodiment of the invention adapted for measuring lung conductivity for monitoring congestive heart failure.
Figure 17.- shows a schematic representation of an embodiment of the invention intended to measure blood pressure in vessels.
Figure 18.- shows two electric diagrams of other preferred embodiments of the implant including a current controlled discharge network, wherein in Figure A the discharge network includes a current controlling device , such as a current-limiting diode (CLD); and in Figure B the discharge network includes a JFET current limiter.

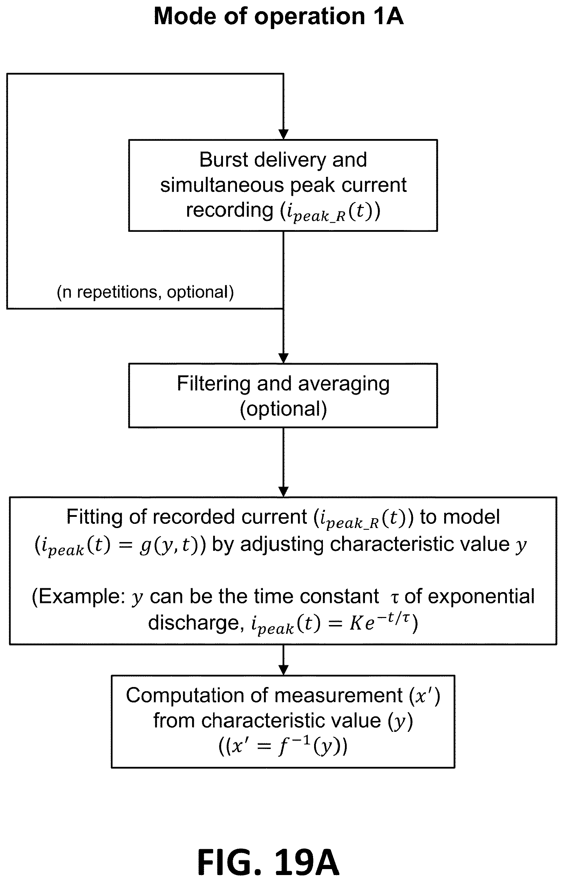

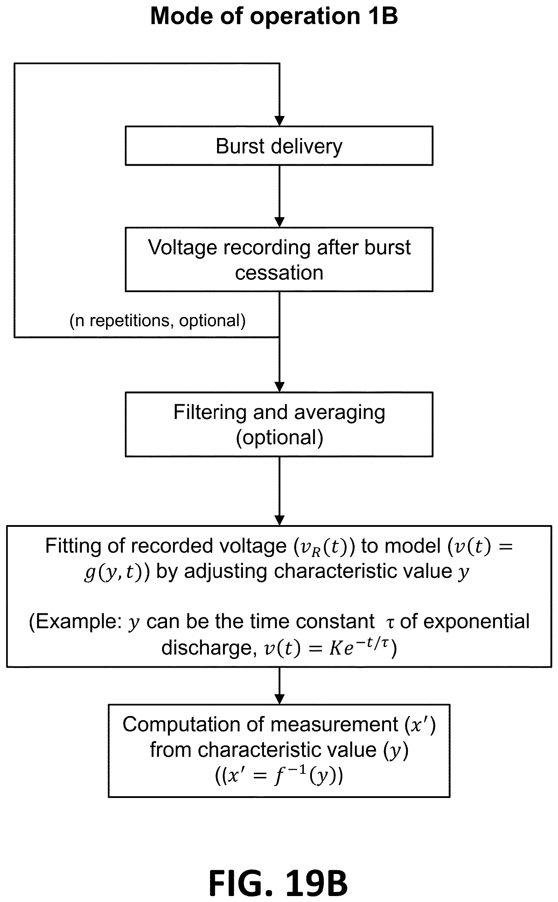

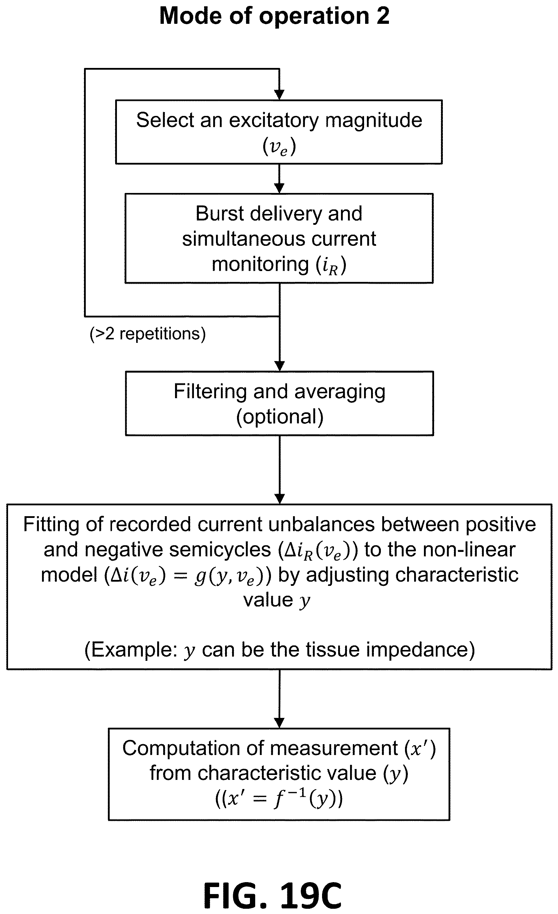

Figure 19.- shows several flow charts illustrating proposed modes of operation for interrogating the implants performed at the remote unit. In particular Figure A illustrates the mode of operation 1A, Figure B illustrates the mode of operation 1B and Figure C illustrates the mode of operation 2.

### Preferred embodiments of the invention

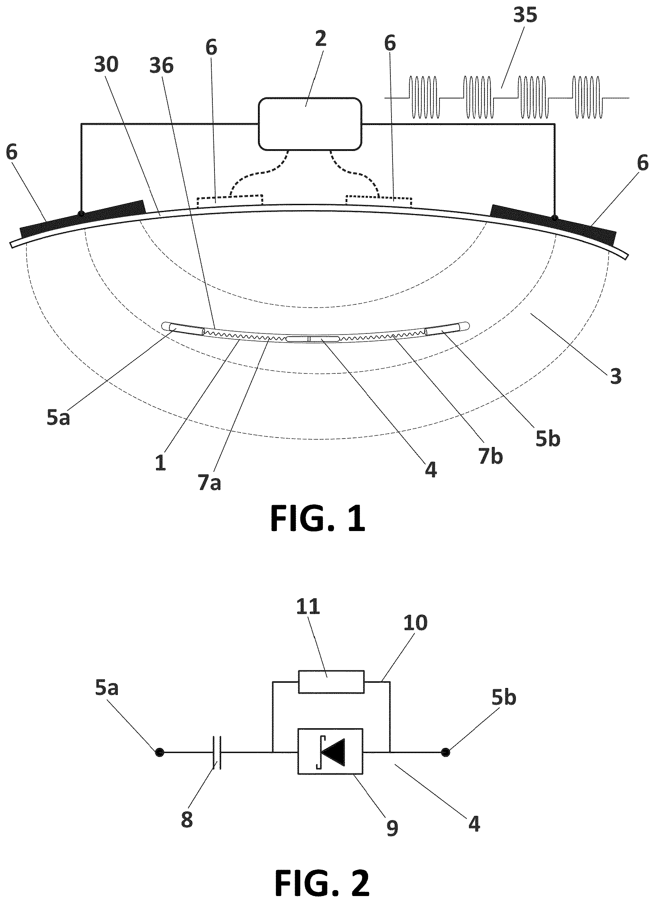

**Figure 1** shows schematically an implantable sensing system according to the invention, comprising an electronic implant (1) implanted in a medium (3) for example a tissue of a living body, and a reading unit (2) adapted to obtain measurements from the implant (1).

The electronic implant (1) comprises an implant circuit (4), at least two electrodes (5a,5b) and two coiled metallic wires (7a,7b) connecting the electrodes (5a,5b) with the implant circuit (4). The implant (1) is configured as an elongated body wherein the implant circuit (4), the two coiled metallic wires (7a,7b) and the electrodes (5a,5b) are linearly arranged, and the electrodes are placed at opposite ends of the elongated body, as shown in **Figure 1****.**

The implant (1) might be constructed as a flexible tubular body (36) made of biocompatible silicone material, and a hermetic capsule housing the implant circuit, wherein the capsule, the electrodes and the coiled metallic wire, reside within the tubular body.

In the embodiment of **Figure 1****,** the reading unit (2) is a battery powered hand-held external unit incorporating electrodes (6) that are manually placed on the skin (30) of a subject over the location of a selected implant (1) to be read. The reading unit (2) generates an interrogation signal comprising at least one burst (35) of alternating current, suitable to reach the implant (1) by volume conduction (represented in **Figure 1** by three dashed lines) through the medium (3).

In other embodiments, a plurality of electrodes (6) of the reading unit (2) are placed on a body region where multiple implants (1) are deployed, and a selective interrogation process is automatically performed by means of electronic switching mechanisms, such as relays or analog multiplexors, such that several implants are sequentially interrogated.

In **Figure 1****,** the reading unit (2) is represented as an external system, however, in other embodiments the reading unit (2), or part of it, is implantable. For example, the reading unit might consist of a subcutaneously implanted sub-unit, adapted to generate bursts (35) of current and for taking measurements, and an external sub-unit adapted for processing data wirelessly transmitted by the implanted sub-unit. The external sub-unit might be adapted for representing measurements and for generating alarms. A conventional smartphone, tablet or similar programmable device, can be adapted for that use.

**Figure 2** shows a basic architecture of the implant circuit (4), that comprises a capacitor (8) and a device of asymmetric conductance (9) capable of rectifying an alternating current (for example a Schottky diode, a LED or a p-n junction of a transistor), such as the capacitor (8) and the device of asymmetric conductance (9) are both connected in series between two electrodes (5a,5b) as shown in **Figure 2****.** The implant circuit (4) further comprises a discharge network (10) connected in parallel with the device of asymmetric conductance (9).

The discharge network (10) includes at least one electrical or electronic component, a resistor (11) in the example of **Figure 2****,** which allows the capacitor discharge bypassing the device of asymmetric conductance (9). As a burst (35) of alternating current reaches the implant (1) by volume conduction, the current is rectified by the device of asymmetric conductance (9) and charges the capacitor (8). When the burst of current ends, the capacitor (8) discharges through the discharge network (10) and through the body medium (3). The capacitor (8) prevents that dc currents flow through the implant (1), as these dc currents would cause irreversible electrochemical reactions at the implant electrodes (5a,5b), that in turn would damage both the electrodes and the living tissues.

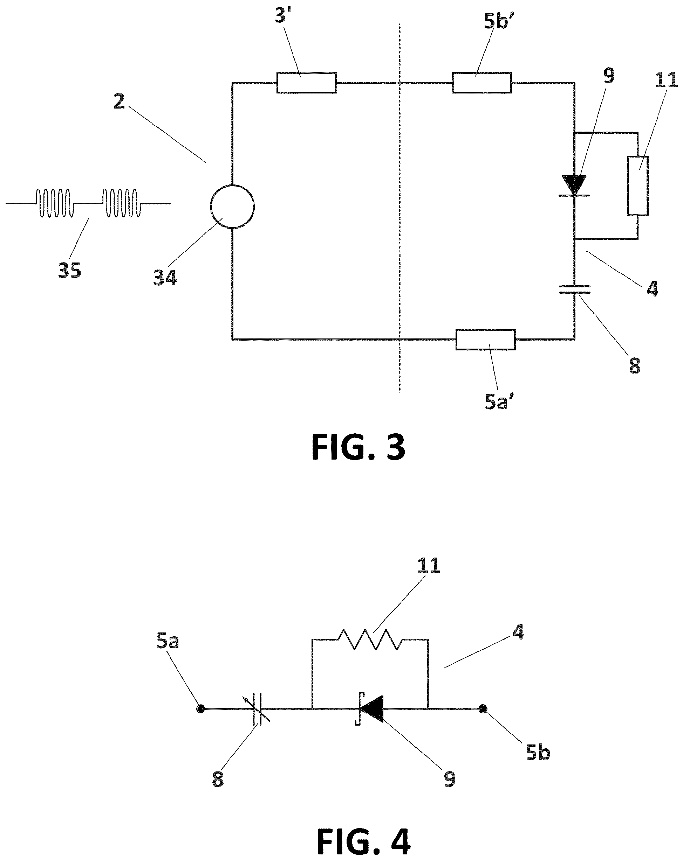

**Figure 3** illustrates the Thévenin equivalent circuit of the medium (3) that surrounds the implant and the electric field. The circuit of **Figure 3** shows the impedance that the implant circuit (4) perceives, including the impedance (5a',5b') of the electrodes (5a,5b), and the equivalent impedance (3') of the medium (3), together with the equivalent voltage source (34) that models the presence of the electric field generated by the current bursts (35). The impedance of the equivalent circuit (*Z_{T}*) corresponds to the impedance across the implant electrodes, that is, the impedance (3') of the medium. This impedance is determined by the passive electrical properties of the medium (i.e. the living tissues) and by the geometry of the implant and its electrodes (5a,5b). In some cases it is acceptable to model it as a resistance.

The behavior of the circuit is determined by the impedance of the medium, both during the delivery of the burst (capacitor charging) and afterwards (capacitor discharging). If the components of the implant circuit (4) have known values, it is possible to measure the impedance of the medium by characterizing the behavior of the circuit using the interrogation methods described below.

The voltage source (34) models the presence of the electric field caused by the delivery of the high frequency current bursts (35). Since the involved media are linear and passive, the waveform of this voltage source will be equivalent to that of the applied current or voltage. The amplitude of this source will be a small fraction of the amplitude of the voltage at the current injecting electrodes of the reading unit (2). Coarsely, the maximum amplitude of the equivalent voltage source (34) will be the amplitude of the electric field at the implant location times the distance between the centers of the implant electrodes. This amplitude will be scaled by the cosine of the angle between the electric field and the direction defined by the implant electrodes.

The impedance (5a',5b') of the electrodes (5a,5b), can be modeled as a non-linear resistance in parallel with a capacitance. The non-linear resistance accounts for current conduction across the electrode by means of electrochemical reactions. For metal electrodes this resistance can be considered to be very large, in the order of hundreds of kiloohms or megaohms, when the voltage across the electrode is below a threshold in the order of some hundreds of millivolts. The capacitance accounts for the capacitance of the so-called double-layer that forms at the interface between the electrode and the medium. This capacitance is in the order of 10 µF/cm² in metal electrodes with a smooth surface.

Two preferred interrogation methods are envisioned for the implants:

### 1.- Operation based on the charging or discharging of the capacitor.

During the delivery of a burst (35) of high frequency electric current, the capacitor (8) is charged by the rectified current generated by the device of asymmetric conductance (9) that produces a change in the passive behavior of the circuit that can be detected by the reading unit (2) by measuring the voltage or current signals. Once the burst (35) ends, the discharging capacitor (8) causes a voltage across the implant electrodes (5a,5b) that can also be detected by the reading unit (2) by measuring the voltage signal picked up with its electrodes (6). In both phases (i.e. during the burst and after the burst), the time course of the signals depends not only on the characteristic values of the device of asymmetric conductance (9) and the capacitor (8) but also on the characteristic values of the mechanism to discharge the capacitor (8) and on the impedance across the circuit terminals, which consists in the series combination of the impedance of the electrodes (5a',5b') and the impedance (3') of the living tissues. The reading unit (2) can produce measurements of the magnitude of interest by processing either the signals during the burst (35) or the signals after the burst (7). The reading unit (2) can also combine results from both stages in order to improve the accuracy of the measurement.

### 2.- Operation based on the non-linear behavior of the circuit.

During the application of the burst (35) of high frequency electric current, the non-linear behavior of the device of asymmetric conductance (9) induces a current unbalance between positive and negative semicycles of the injected signal. This current unbalance depends not only on the electrical characteristics of the implant components and the living tissue but also on the applied voltage magnitude. The reading unit (2), by performing and processing voltage and current measurements using different excitatory magnitudes, can characterize the non-linear behavior of the device of asymmetric conductance (9) and produce measurements of the magnitudes of interest.

In a preferred embodiment the system is used to measure the electrical impedance or conductance of the tissues surrounding the implant. The implant circuit used for measuring the impedance of surrounding tissues can simply consist of a capacitor in series with the parallel combination of a diode and a resistor.

From the measured impedance it is possible to extract the impedivity of the medium by scaling the impedance according to the cell constant (*k*). The cell constant is a geometrical factor, typically expressed in units of (m⁻¹) but also sometimes expressed in units of (m) that can be obtained by numerical methods or that can be obtained by measuring a medium of known admittivity (i.e. by calibration). If the measured impedance can be approximated as a resistance (*Rₘ*), then it will be possible to use the cell constant to extract the conductivity (*σ* = *k*/*Rₘ*) or the resistivity of the medium (*ρ* = *Rₘ*/*k*).

If the interrogation method is based on discharging the implant capacitor (see below), and the impedance across the implant electrodes can be modeled as a resistance (*R_{T}*), then the time constant (*τ_{D}*) will correspond to *τ_{D} = C*(*R_{T} + R_{D}*) where *C* is the capacitance of the capacitor of the implant and *R_{D}* is the resistance of the discharging element of the implant. Hence the resistance measurement (*Rₘ*) will be simply calculated as: *Rₘ* = (*τ_{D}*/*C*) *- R_{D}.*

Different magnitudes can be transduced into capacitance. For instance, a large group of pressure sensors is based on isolated conductors that deform under pressure thus becoming closer and hence increasing their mutual capacitance
**Figure 4** shows a preferred embodiment with the same circuit architecture of **Figure 2****,** but adapted for performing measurements based on sensors whose capacitance depends on the magnitude of interest. In this embodiment, the electrical or electronic component of the discharge network (10) is also a resistor (11) of fixed value, and the capacitor (8) is a transducer whose capacitance is variable depending on a physical and/or chemical condition of the medium (3). Preferably, the capacitor (8) is a pressure capacitor, whose capacitance depends on the pressure applied to a part of the capacitor. The device of asymmetric conductance (9) is a Schottky diode, that is preferred due to its low forward voltage drop that allows shorter implants or lower electric field amplitudes.

Since the transducer capacitance is in series with the uncontrollable capacitance of the electrodes (see **Figure 3**), it is required that the capacitance value of the transducer (8) is significantly smaller than that of the electrodes (5a,5b), so that the capacitance value of the series combination is dominated by that of the transducer.

The capacitance of metallic electrodes with a smooth surface is in the order of 10 µF/cm². Therefore, assuming that the implant electrodes (5a,5b) have a surface area in the order of 1 mm², the above requirement would imply that capacitance value of the transducer has to be much lower than 100 nF.

On the other hand, the capacitance of the transducer needs to be much larger than the possible parasitic capacitances in the implant. These parasitic capacitances can be expected to have values in the order of picofarads.

Therefore, from the above it can be concluded that, in a preferred embodiment of the implant (1) consisting of a thin and flexible elongated body, with a length ranging from a few millimeters to a very few centimeters, and with two electrodes (5a,5b) at opposite ends, the optimal values for the capacitance of the transducer will be within the range: 10 pF to 10 nF.

Similarly, when the interrogation method is based on the discharge of the capacitance of the transducer, it will be advantageous to select a discharge network (10) with a resistance value much higher than the impedance magnitude of the tissues during the discharge. For soft living tissues and the dimensions of the preferred embodiment of the preceding paragraph, those impedance magnitudes will range from about 100 Ω to 10 kΩ. And, therefore, it can be anticipated that it will be optimal to select resistors with a resistance between 1 kΩ to 10 MΩ. Larger resistances probably will not be optimal because: 1- they will be comparable to parasitic resistances in the implant and 2- they will cause long charging and discharging times that will lengthen the measurement time.

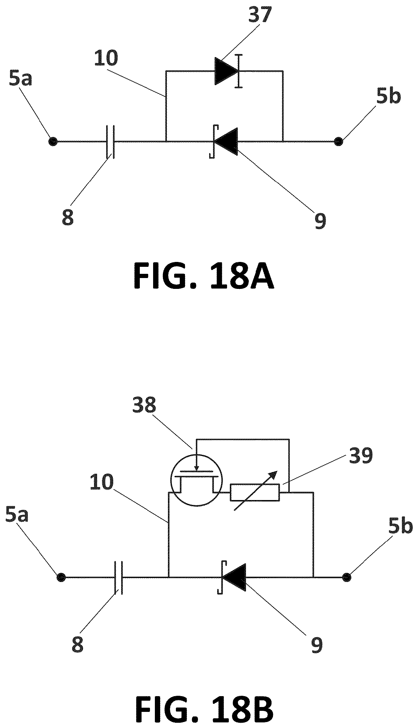

Alternatively, instead of using a discharge network with a controlled resistance, it can be implemented a discharge network of controlled current. In this preferred embodiment, the electric or electronic component of the discharge network (10) is a current controlling device, such as a current-limiting diode (CLD) (37) (represented in **Figure 18A****)** or a JFET current limiter (38) that controls the discharge current of the capacitor and renders it independent of the impedance of the medium. That current controlling device may be of nominal current like the CLD (37), or may be configured to depend on a measurand. For instance, in the latter case (represented in **Figure 18B**), the resistor (39) in a JFET current limiter (38) may be a resistive transducer.

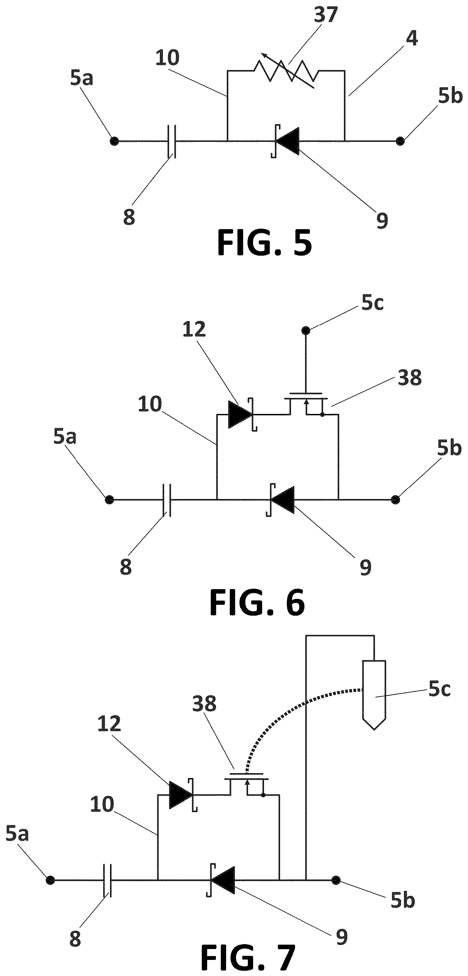

**Figure 5** shows another preferred embodiment with the same circuit architecture of **Figure 2****,** but adapted for performing measurements based on sensors whose resistance depends on the magnitude of interest. In the embodiment of **Figure 5****,** the capacitor (8) has a fixed nominal capacitance, the device of asymmetric conductance (9) is a Schottky diode, and the electric or electronic component of the discharge network (10) is a resistive transducer (37), for instance a thermistor or a Light Dependent Resistor (LDRs). Thermistors are known two-terminal semiconductor components that non-linearly transduce temperature into resistance.

**Figure 6** shows an embodiment of the implant for measurement of biopotentials, wherein the capacitor (8) has a fixed nominal capacitance, the device of asymmetric conductance (9) is a Schottky diode and the electronic component is a transducer configured as a transistor (38) whose gate or base terminal is in contact with the medium (3) through a third electrode (5c). The conductance of the transistor (38) depends on the voltage at that third electrode (5c) with respect the other two electrode (5a, 5b). Preferably, the discharge network (10) also includes an auxiliary diode (12) (also a Schottky diode) connected in series with the transistor (38) to prevent conduction when the main diode (9) is forward biased. The anode of the auxiliary diode (12) is connected with the cathode of the diode (9) and one terminal of the capacitor (8), the cathode of the auxiliary diode (12) is connected with the collector or drain of the transistor (38), and the source or emitter of the transistor (38) is connected with the anode of the diode (9) and with anode electrode (5b). The other terminal of the capacitor (8) is connected with the cathode electrode (5a).

Preferably, the transistor (38) is a MOS transistor or a BJT.

**Figure 7** is a variation of the circuit of **Figure 6** for sensing chemical species instead of voltage. In **Figure 7** the transistor (38) is a ChemFet transistor or an Ion Selective Field Effect Transistor (ISFET) for measuring concentrations of chemical species. In this embodiment, the gate of the transistor is directly in contact with the medium and a third electrode (5c) used as reference electrode to stablish ionic conductance (represented as a dotted line in **Figure 7**) between the gate and the third electrode (5c). This results in a dependency of the drain-source current on the concentration of the chemical species.

Selectivity to the ion of interest is determined by the material of the gate and of its coatings. Membranes are implemented on the gate to creative selective ISFETs for a wide range of ions (e.g. K⁺, Na⁺ and Cl⁻ ) and other chemical species (e.g. glucose).

Other preferred embodiments of the invention are based on optoelectronic components (e.g. light dependent resistors (LDRs), photodiodes and phototransistors) whose conductance depends on the received light intensity. Some electronic components, such as light emitting diodes (LEDs), are capable of producing light. All these components are readily integrated in the present invention, to implement implants sensitive to the optical characteristics of the medium (3) or the optical characteristic of a material of the implant that can be in contact with the medium (3).

The material to be optically characterized can be the tissue surrounding the implant (e.g. for pulse oximetry) or a material of the implant. This second option is of particular relevance as there are materials that can transduce a wide range of magnitudes of clinical interest (e.g. chemical concentration of specific chemical species) into optical properties (e.g. fluorescence or phosphorescence characteristics).

Therefore, in another preferred embodiment of the invention, the device of asymmetric conductance (9) and/or the electrical or electronic component of the discharge network (10), is a LED, or a light sensitive semiconductor, for example: a photodiode, or a phototransistor whose conductance depends on the received light.

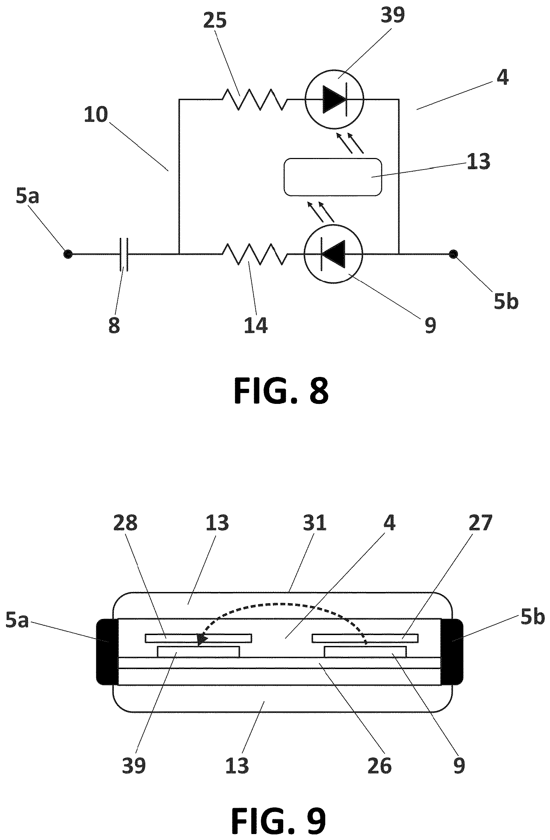

In this range of optical implants, **Figure 8** shows another preferred embodiment of the invention configured for transducing the phosphorescence of an optical reactive material into the conductance of the discharging network. In the embodiment of **Figure 8****,** the capacitor (8) has a fixed nominal capacitance and the device of asymmetric conductance and the electronic component of the discharge network (10), are constructed in combination as a transducer incorporating an optical reactive material (13), preferably a fluorescence or phosphorescence reactive material, that is arranged in the implant to be in contact with the medium (3).

More in detail, the device of asymmetric conductance is a light emitting semiconductor device (9), for example a Light Emitting Diode (LED) connected in series with a resistance (14). The electronic component is a light sensitive (receiving) conductive device connected in series with a second resistance (25). Preferably in this embodiment, the light sensitive conductive device is a photodiode (39), but it could also consist of an LDR, a phototransistor or a similar component. The second resistance (25) and the photodiode (39) are connected in parallel with the first resistance (14) and the LED (9), such that the cathode of the photodiode (39) is connected with the anode of the LED (9), and one terminal of the resistances (14,25) are connected with one terminal of the capacitor (8), as shown in **Figure 8****.**

The optical reactive material (13) is arranged in the implant, to receive light from the LED (9) during the capacitor charge, and to irradiate light to the photodiode (39), such as when a current burst ends, the capacitor (8) discharges through the photodiode (39) while this receives light from the optical reactive material (13) due to its fluorescence or phosphorescence property. The optical reactive material (13) is selected, such that its fluorescence or phosphorescence property, is variable depending on a physical or chemical condition of the medium.

**Figure 9** shows a preferred example of constructing the electronic implant of **Figure 8****.** In **Figure 9** the implant comprises a capsule (31) formed at least in part with the optical reactive material (13). The electronic implant circuit (4) is housed within the capsule (31), and comprises a mounting substrate (26) such as a Printed Circuit Board, and the light emitting semiconductor device (9), the light sensitive conductive device (39), and the resistances (14, 25), are mounted on the substrate (26). The optical reactive material (13) is any known material that can transduce a magnitude of interest (e.g. oxygen concentration) into an optical property (e.g. phosphorescence). Such material could, for instance, consist of a biocompatible hydrogel with phosphorescent dyes with oxygen sensitivity (e.g. Zn²⁺ porphyrins).

The circuit depicted in **Figure 8** is adequate for optical reactive materials exhibiting phosphorescence but, depending on the interrogation method, it may not be adequate for materials not exhibiting phosphorescence because the LED only emits light when the capacitor is being charged, and not when it discharges. A solution to that consists in intercalating a phosphorescent material to illuminate the optical reactive material during the discharge, that is, after the LED ceases emitting light.

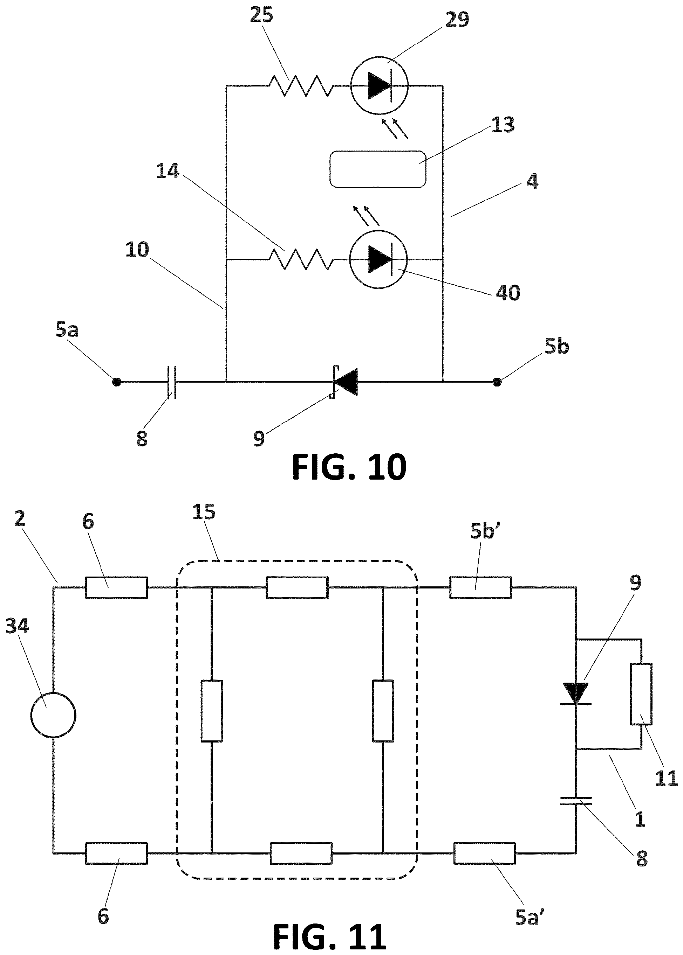

An alternative solution consists in implementing circuits that emit light during the discharge. An example of such circuits is illustrated in **Figure 10****,** wherein the capacitor (8) has a fixed nominal capacitance, the device of asymmetric conductance (9) is a Schottky diode, and wherein the discharge network (10) comprises a light emitting semiconductor device preferably a LED (40) connected in series with a first resistance (14), and a light sensitive conductive device is for example a photodiode (29) connected in series with a second resistance (25). The second resistance (25) and the photodiode (29) are connected in parallel with the first resistance (14) and the LED (40) and in parallel with the Schottky diode (9), such that the cathode of the photodiode (29) is connected with the cathode of the LED (40), and one terminal of the resistances (14,25) are connected with one terminal of the capacitor (8) and with the cathode of the Schottky diode (9), as shown in the figure. The optical material (13) is arranged to transmit light from the LED (40) to the photodiode (29). The capacitor (8) discharges through the LED (40) so as to emit light during the capacitor discharge. The optical material (13) is a transmitting, reflecting, or refractive optical reactive material. During the discharge, the LED (40) illuminates the transducer material (13) which in turns illuminates - by reflection, transmission, refraction or fluorescence - the photodiode (29) thus determining the discharging rate.

The implant of **Figure 10** can be constructed as the embodiment of **Figure 8****.**

In order to increase the selectivity of the above implants based on optoelectronic elements, and in particular when it is characterized the fluorescence of the transducer material, it may be advantageous to intercalate optical filters or diffraction grids (27,28) placed on the photoemitter and on the photodetector (photodiode) or as shown in **Figure 9** **and** **Figure 10****,** so as to select specific light wavelengths or bands of operation.

Since the optical characteristics of a piece of material not only depend on its intrinsic optical properties but also on its geometry, the above circuits can also be employed to sense changes of geometry. For instance, the transducer material in **Figure 9** can consist in a soft material that compresses under mechanical pressure thus modifying its optical transmissivity between the light emitter and the detector, and hence acting as a pressure transducer.

**Figure 11** facilitates comprehension of the different proposed modes of operation, and illustrates a possible implant architecture according to the invention together with the impedance models for the implant (1) and the reading unit electrodes (6), and an impedance two-port model (15) for the living tissues that couples the reading unit (2) to the implant (1).

Two fundamental modes of operation are envisioned for interrogating the implants:

### 1. Operation based on the charging or discharging of the capacitor.

### 1.A. Operation based on monitoring the load seen by the reading unit during the delivery of the burst:

As the capacitor (8) of the implant (1) charges during the delivery of the burst, the ratio between the output voltage amplitude and the output current amplitude of the reading unit (2) increases because, during the semicycles in which the diode (9) is forward biased, the network branch corresponding to the implant progressively draws less current. If the waveform generated by the reading unit (2) is voltage controlled, this is perceived by the voltage generator (34) as a subtle decrease in the amplitude of the current (*iₚₑₐₖ*(*t*)) for the semicycles in which the diode (9) is forward biased. This signal, *iₚₑₐₖ*(*t*), can be extracted at the reading unit (2) with a peak detector. While the absolute magnitude of *iₚₑₐₖ*(*t*) depends on the coupling factor between the reading unit electrodes (6) and the implant electrodes (5a,5b), the time course of relative changes in *iₚₑₐₖ*(*t*) does not.

Since the coupling factor is an uncontrollable parameter as it depends on the distance between the reading unit and the implant, it is preferred to base the interrogation on analyzing the time course of relative changes in *iₚₑₐₖ*(*t*). The time course of relative changes in *iₚₑₐₖ*(*t*) depends on the impedance of the tissues as seen by the implant (*Z_{T}*), the impedance of the implant electrodes (*Z_{E}*), the capacitance (*Cᵢ*) of the implant capacitor, the resistance (*R_{D}*) of the discharging element of the implant and the characteristics of the diode.

The time course of relative changes in *iₚₑₐₖ*(*t*) can be modelled analytically or numerically (e.g. by SPICE simulations) to characterize its dependence on the referred elements. Then, contrasting such characterization with the recorded time course of relative changes in *iₚₑₐₖ*(*t*), the reading unit can compute the characterization of an unknown element if the other ones are known. In the specific case in which the equivalent impedance of the tissues can be approximated to behave as a resistance (i.e. *Z_{T}* = *R_{T}*) and the impedance of the implant electrodes can be neglected (*Z_{E}*= 0), *iₚₑₐₖ*(*t*) will follow an exponential time decay whose time constant (*τ*) will be computable by the reading unit as described in the operation mode 1.B. Then, if the capacitance (*Cᵢ*) of the capacitor and the resistance (*R_{D}*) of the discharging element are fixed and known, the reading unit will be able to compute the value of the resistance of the medium.

If the waveform generated by the reading unit (2) is current controlled instead of voltage controlled, analogous computing procedures to those described above can be carried out by the reading unit (2) by processing the subtle increases in the voltage amplitude at the semicycles in which the diode is forward biased (*vₚₑₐₖ*(*t*)).

Since the subtle changes in either current or voltage are overlapped on a large signal, the implementation of the method requires detection and processing with a very large dynamic range.

Another inherent limitation of this mode of operation is that it is only useful to measure the resistance or the impedance of the tissues. That is, it is not useful to independently measure neither the capacitance of the implant capacitor nor the resistance of the discharge network.

### 1.B. Operation based on monitoring the voltage produced during the discharge of the capacitor:

The capacitor (8) of the implant (1), which is charged during the delivery of the burst, discharges after burst cessation through the discharge network (10), the implant electrodes (5a,5b) and the tissues surrounding the implant. This produces a decaying voltage across the implant electrodes that can be sensed distantly by pairs of electrodes in contact with the tissues. In a preferred embodiment, this voltage is sensed across the electrodes (6) of the reading unit (2). By characterizing the sensed waveform, it is possible to compute the capacitance of the implant capacitor (8), the resistance (11) of the discharging element of the implant circuit or the impedance of the tissues. In particular, if the impedance of the tissues can be modeled as a resistance (i.e. *Z_{T}* = *R_{T}*) and the impedance of the implant electrodes is neglected, the sensed voltage will be: ${v}_{sensed} \left(t\right) = α {V}_{0} {e}^{- t / τ} ; τ = {C}_{i} \left({R}_{D}+{R}_{T}\right)$ where *α* is the coupling factor between the implant electrodes and the sensing electrodes, *V*₀ is the initial voltage across the implant electrodes right after burst cessation, *Cᵢ* is the capacitance of the implant capacitor and *R_{D}* is the resistance of the discharging element of the implant.

From the above it is obvious that any of the values *Cᵢ, R_{T}* and *R_{D}* can be obtained if the value of the time constant (*τ*) is computed and the other values are known.

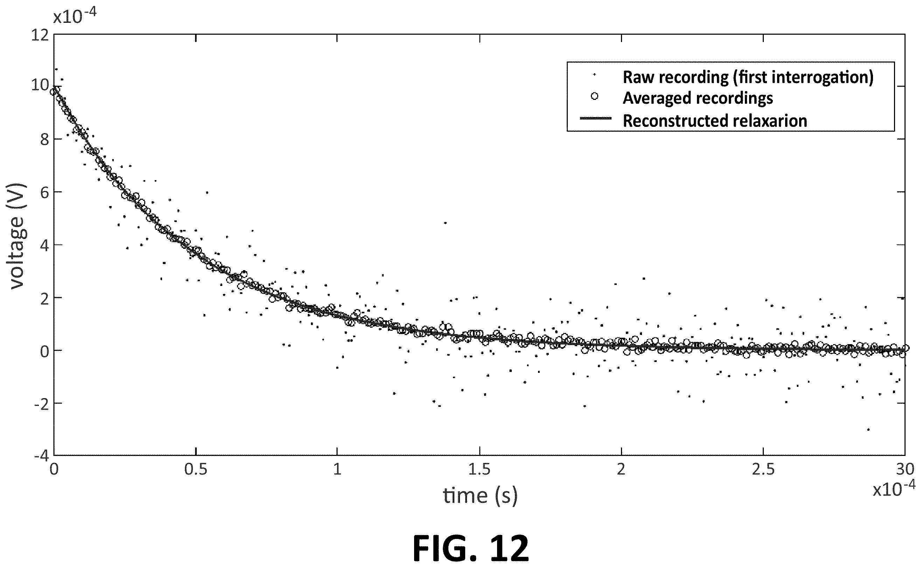

From the sensed voltage (*v_{sensed}*(*t*)) it is possible to extract the value of the time constant (*τ*) by different known methods. In a preferred embodiment of the invention, the time constant is obtained by fitting the average of multiple recordings of the sensed voltage to a generic exponential decay function (*y*(*t*) = *Ae^{-γt}*) by using least squares fitting. An example of the result of this operation is represented in the graph of **Figure 12****.** This procedure to obtain an estimation of the time constant provides good results under the presence of noise and interferences.

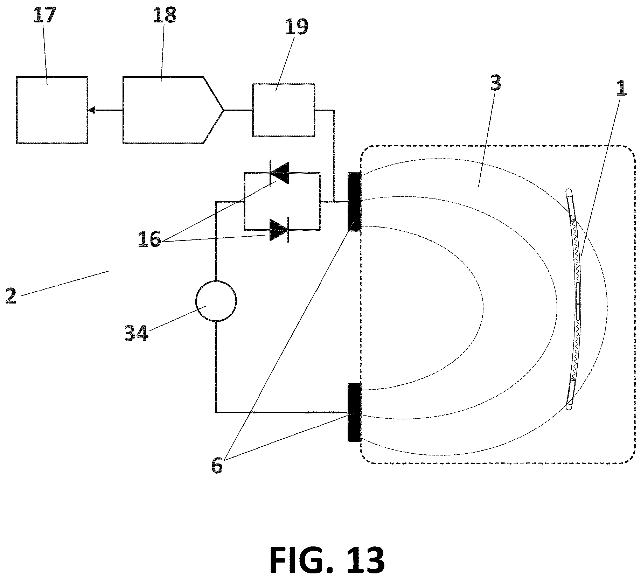

**Figure 13** shows a preferred embodiment of the system of the invention incorporating an implant (1) deployed in a medium (3), and a reading unit (2) to interrogate the implant (1). The reading unit (2) is represented by its electric diagram that allows recording the sensed voltage (*v_{sensed}*(*t*)) with the same electrode (6) pair used for delivering the bursts. The burst is generated by a voltage generator (34) of relatively high amplitude, for example above 5V. After the interrogation signal terminates, the voltage generator (34) disconnects from the electrodes (6) so that it does not short-circuit them.

In the embodiment of **Figure 13****,** such disconnection is performed by two diodes (16) in antiparallel connection: the diodes (16) allow passage of high magnitude signals during the delivery of the interrogation signal, but behave as an open circuit for the much smaller sensed voltage, which will have a voltage amplitude below 100 mV. A low-pass filter (19) is used to suppress noise and interferences and to prevent saturation of posterior stages. The sensed voltage (*v_{sensed}*(*t*)) is captured with an analog-to-digital converter (18) and later processed by a control and processing module (17) to obtain the time constant as described above.

### 2. Operation based on the non-linear behavior of the circuit

During the application of the burst of high frequency electric current, the non-linear behavior of the diode (9) induces a current unbalance between positive and negative semicycles of the injected signal. This current unbalance depends not only on the electrical characteristics of the implant elements and the living tissues but also on the applied voltage magnitude. The reading unit (2), by performing and processing voltage and current measurements using different excitatory magnitudes, can obtain a set of independent equations from which it can compute the characteristic values of the elements of the system and hence produce measurements of the magnitudes of interest. Here it is illustrated the procedure by detailing a simplified example.

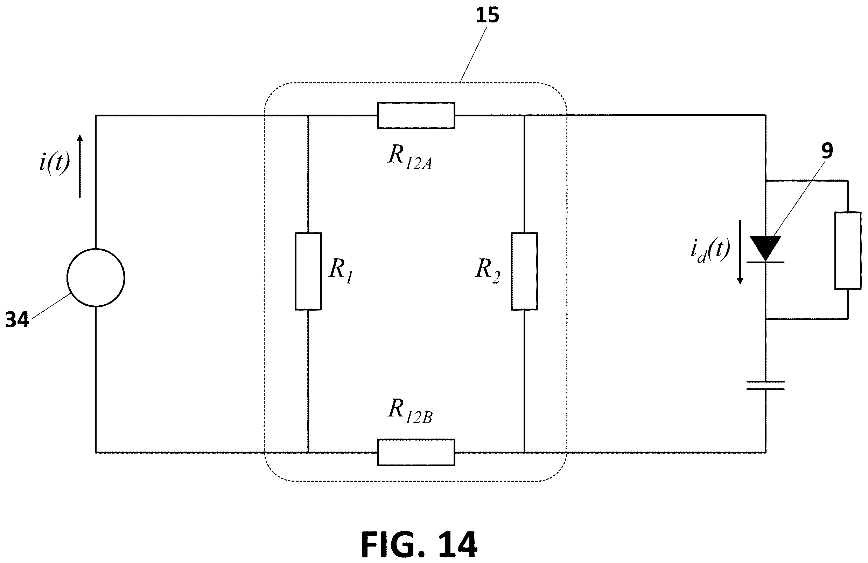

**Figure 14** shows the electrical representation of the system used to illustrate the procedure where the living tissues model (15). At high frequencies, living tissues can be approximated to behave as resistances and the electrode interface impedances can be neglected.

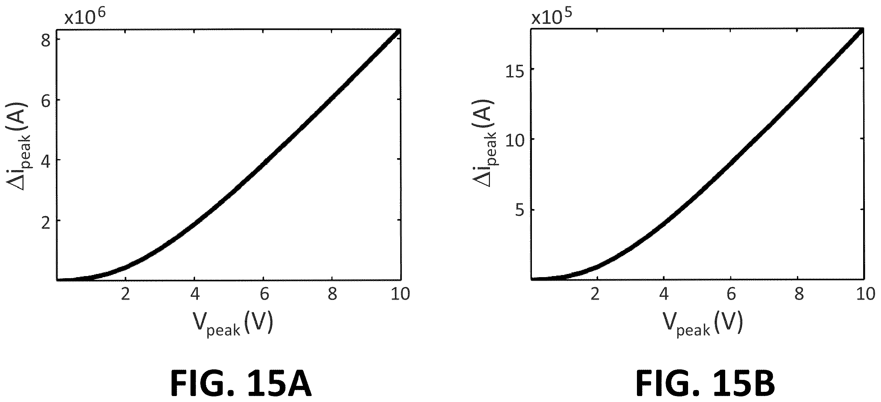

For the illustration scenario (**Figure 14**), in the case of a voltage controlled reading unit (2) that delivers sinusoidal bursts, the output current amplitude (*iₚₑₐₖ*(*t*)) during the semicycles in which the diode is forward biased (*iₚₑₐₖ*|₊) shows larger values than during the complementary semicycles (*iₚₑₐₖ*|₋). This results in a current difference between both semicycles (Δ*iₚₑₐₖ*(*t*)). Because of the non-linear behavior of the diode (9), the current unbalance between semicycles at the reading unit (2) depends on the voltage amplitude (*vₚₑₐₖ*(*t*)) **(****Figure 15a****).** The same non-linear behavior is present at the implant side **(****Figure 15b****).**

Since the non-linear behavior of the diode (9) can be well characterized, by measuring the current unbalance between semicycles at the reading unit (2) for different applied voltages, it is possible to detect the non-linear behavior which indirectly determines the current circulating through the implant.

The reading unit (2) is able to compute the value of the resistance values of the living tissue port model (15) based on the current peak unbalance (Δ*iₚₑₐₖ*) and the characterized non-linear behavior of the implant. This can be readily illustrated with the proposed example. Considering very short bursts, the implant can be simplified as a single diode (9) with an idealized behavior: it causes a constant forward voltage drop (*v_{f}*) when the diode is forward biased and completely blocks current when it is reverse biased. Then: $Δ {i}_{peak} \left({v}_{peak}\right) = {i}_{peak} {\left|- +{i}_{peak}\right|}_{-}$ ${i}_{peak} \left|= +\frac{{v}_{peak}}{R 1}+\frac{{v}_{peak} - {v}_{f}}{{R}_{12}}\right.$ ${i}_{peak} \left|= -\frac{{v}_{peak}}{{R}_{1}}+\frac{{v}_{peak}}{{R}_{12} + {R}_{2}}\right.$ $Δ {i}_{peak} \left({v}_{peak}\right) = \frac{{v}_{peak} - {v}_{f}}{{R}_{12}} - \frac{{v}_{peak}}{{R}_{12} + {R}_{2}}$

Where ${R}_{12} = {R}_{12 A} + {R}_{12 B}$

Therefore, taking advantage of the non-linearity of the diode, by performing additional measurements at different voltage magnitudes, non-redundant (i.e. independent) equations can be obtained that allow solving the unknowns of the system from which measurements can be produced. $Δ i \left({v}_{1}\right) = \frac{{v}_{1} - {v}_{f}}{{R}_{12}} - \frac{{v}_{1}}{{R}_{12} + {R}_{2}} \to \frac{1}{{R}_{12} + {R}_{2}} = \frac{\frac{{v}_{1} - {v}_{f}}{{R}_{12}} - Δ i \left({v}_{1}\right)}{{v}_{1}}$ $Δ i \left({v}_{2}\right) = \frac{{v}_{2} - {v}_{f}}{{R}_{12}} - \frac{{v}_{2}}{{R}_{12} + {R}_{2}} \to \frac{1}{{R}_{12} + {R}_{2}} = \frac{\frac{{v}_{2} - {v}_{f}}{{R}_{12}} - Δ i \left({v}_{2}\right)}{{v}_{2}}$ ${R}_{12} = \frac{{v}_{2} ⋅ \left({v}_{1}-{v}_{f}\right) - {v}_{1} \left({v}_{2}-{v}_{f}\right)}{Δ i \left({v}_{1}\right) ⋅ {v}_{2} - Δ i \left({v}_{2}\right) ⋅ {v}_{1}}$

In case of current controlled reading unit, analogous computing procedures to those described above can be carried out by replacing current to voltage terms and vice versa.

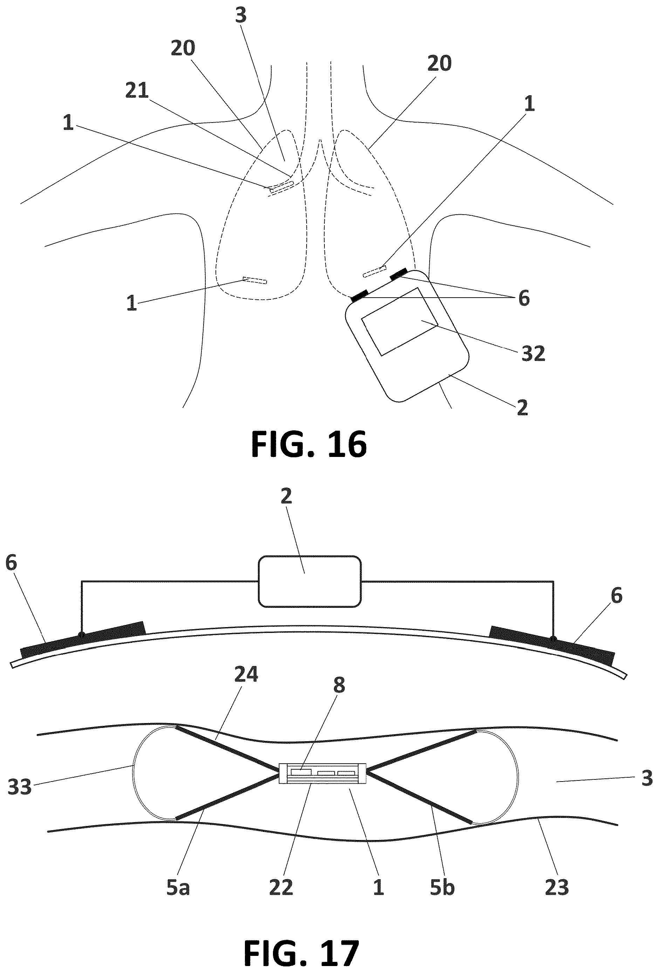

**Figure 16** also illustrates a practical application of the system of the invention, intended to measure lung impedance for early detection of worsening heart failure. In this embodiment, implants (1) according to the invention can be deployed at different possible locations either in the lungs (20) or in nearby tissues to monitor the conductivity of the lungs.

Pulmonary edema, that is, accumulation of fluids in the lungs, occurs during worsening heart failure. Since such accumulation of fluids causes an increase in the conductivity of the lungs, the measurements provided by the implants (1) can be used to determine when heart failure is worsening.

In this embodiment of the invention, the implants (1) are deployed at locations that will result in a higher sensitivity to the conductivity of the lungs than that offered by systems utilizing endovascular electrodes. For instance, the implants (1) can be deployed at the intercostal spaces. There, implants can be easily and safely deployed within the intercostal muscles using percutaneous procedures (i.e. injection). Alternatively, for maximizing sensitivity, the implants can be deployed in the lungs. In the lungs, the implants can be deployed within the parenchyma using a percutaneous procedure or within the airways (i.e. bronchi and bronchioles (21)) through bronchoscopy. In the latter case, for facilitating fixation, the implants (1) are shaped as pulmonary stents.

A reading unit (2) for interrogating the implant here is a battery-powered hand-held unit integrating skin electrodes (6) and a display (32), that the patient positions on his or her skin close to the implant to be interrogated. It can also consist in battery-powered unit shaped as a pod or capsule that is fixed on the skin over the location of the implant, for instance, by a fastener on sticking plaster, and that stores measurements in memory or transmits those measurements by radio to a nearby computerized device such as a smartphone.

**Figure 17** illustrates an alternative embodiment of the implant (1) suitable to be deployed inside an artery or a vein (23) for measuring blood pressure. The implant (1) comprises a capsule (22) having at least a part made of a flexible material that allows pressure transmission from the exterior (caused by blood pressure) to the capsule interior. For example, the flexibility and hermeticity of the capsule (22) can be obtained with a tubular capsule of thin metallic walls of thickness < 0.5 mm.

The implant circuit (4) is hermetically housed within the capsule (22), and it can consist in any of the configurations described above suitable for performing measurements with a pressure sensor.

The implant further comprises two electrodes (5a,5b) passing through the capsule (22) and connected to the implant circuit (4), wherein each electrode (5a,5b) is a flexible structure configured to anchor the implant to an artery or vein.

The connection between the electrodes (5a,5b) and the circuit (4) can be performed through hermetic feedthroughs to ensure the hermeticity of the capsule (22). Each electrode is a flexible wire conformed as a loop. An inner part of the electrodes (5a,5b) is covered by an insulating material (24), and an outer part (33) of the electrodes (5a,5b) is exposed to the medium, flowing blood in this case. This configuration for the implant electrodes (5a,5b) facilitates implant deployment, anchorage and extraction. For deployment, the loops can be readily folded within a catheter for enabling minimally invasive implantation through catherization. After deployment, the loops will unfold due to their flexibility or because of shape memory if the wire is made of a metal exhibiting such property such as nitinol and, by pressing the vessel walls, will anchor the implant within the vessel. If required, for minimally invasive implant extraction, the implant can be extracted with a catheter by pulling out with a hook one of the two loops of the implant.

The pressure sensor can be a capacitive pressure sensor. In this case the circuitry of the implant can consist in the circuit illustrated in **Figure 4** for performing measurements with capacitance-based sensors.

The capacitive pressure sensor does not necessarily have to consist in a stand-alone sensor. It can consist in a capacitive pressure sensor formed by using the capsule wall as one of the electrodes of a capacitor. For instance, a dielectric (e.g. air) can be sandwiched within the coaxial structure formed by the semi-flexible capsule wall and an inner, and more rigid, metallic tube or cylinder to form a capacitor whose capacitance increases when the distance between the two metallic parts decreases as the result of pressure.

## Claims

1. A sensing system being configured to measure physical and/or chemical parameters of a living body, the system comprising:
- at least one implant (1), configured to be implanted in a medium (3) of the living body, said implant (1) comprising an electronic circuit (4) and at least two electrodes (5a,5b) connected to the electronic circuit (4), wherein the electronic circuit (4) comprises a capacitor (8) and a device of asymmetric conductance (9) both connected in series between the two electrodes (5a,5b), the electronic circuit (4) further comprising a discharge network (10) connected in parallel with the device of asymmetric conductance (9), the discharge network (10) being configured to discharge the capacitor (8) by bypassing the device of asymmetric conductance (9), wherein the discharge network (10) comprises at least one electrical or electronic component (11), wherein the capacitor (8) and/or the device of asymmetric conductance (9) and/or the electrical or electronic component (11) is a transducer comprising one or more characteristic values which are configured to vary depending on a physical and/or chemical condition of the medium (3) when the implant (1) is deployed in the medium (3); and
- a reading unit (2) configured to read the electronic implant (1) when the implant (1) is deployed in the medium (3) of the living body, wherein the reading unit (2) comprises two or more reading unit electrodes (6), an alternating voltage generator (34) to generate an alternating voltage across the reading unit electrodes (6), and a control and processing module (17), wherein the reading unit electrodes (6) are configured to be placed in contact with the skin of the living body, and wherein the reading unit (2) is configured for:
• emitting an interrogation signal via the reading unit electrodes (6), said interrogation signal comprising at least one burst (35) of an alternating current suitable to reach the implant (1) by volume conduction through the medium (3);
• detecting, in the reading unit electrodes (6) or in the electrodes (5a, 5b) connected to the electronic circuit (4), resulting voltage and/or current signals from the implant (1), generated as a response to the interrogation signal;
• processing, with the control and processing module (17), said detected signals to infer the one or more characteristic values of the transducer; and
• obtaining, from the one or more inferred characteristic values of the transducer, a value of the physical and/or chemical parameters of the living body to be measured.

2. A sensing system according to claim 1, wherein the reading unit (2) is adapted to emit bursts (35) of alternating current at a frequency between 100 kHz and 100 MHz, with bursts (35) duration between 0.1 µs and 10 ms, and a repetition frequency between 0 Hz and 100 kHz.

3. A sensing system according to any of the claims 1 or 2, wherein the implant (1) comprises an elongated and flexible body (36) made of an electrically isolating material, and wherein the electronic circuit (4) is housed within the body, and wherein the implant (1) further comprises two metallic electrodes (5a,5b) at the opposite ends of the body which are electrically connected to the electronic circuit, and wherein optionally the length of the implant is within the range 0.5 cm - 5 cm.

4. A sensing system according to any of the claims 1 to 3, wherein the discharge network (10) of the implant (1) comprises a current controlling device for controlling the discharge current of the capacitor (8) and makes the discharge process independent of the impedance of the medium (3') and of the electrodes (5a',5b').

5. A sensing system according to any of the preceding claims, wherein the device of asymmetric conductance (9) is a diode (preferably a Schottky diode or a LED), a p-n junction of a transistor or a smart diode.

6. A sensing system according to any of the claims 1 to 5, wherein the electrical or electronic component (11) of the discharge network is a resistor of a given nominal value, and wherein the capacitor (8) is a transducer whose capacitance is variable depending on a physical and/or chemical condition of the medium (3).

7. A sensing system according to any of the claims 1 to 5, wherein the capacitor (8) has a given nominal capacitance, and wherein the electronic component (11) of the discharge network is a resistive transducer.

8. A sensing system according to any of the claims 1 to 5, wherein the capacitance of the capacitor (8) is within the range 10 pF to 10 nF, and wherein the resistance of the discharge network (10) is within the range 1 kΩ to 10 MΩ.

9. A sensing system according to any of the claims 1 to 5, suitable for measuring biopotentials, wherein the capacitor (8) has a given nominal capacitance, and wherein the discharge network (10) comprises a transistor (38), whose gate or base terminal is arranged to be in contact with the medium (3) by means of a third electrode (5c), such as the conductance of the transistor depends on the voltage at the third electrode (5c).

10. A sensing system according to any of the claims 1 to 5, suitable for measuring chemical species, wherein the capacitor (8) has a given nominal capacitance, and wherein the discharge network (10) comprises a ChemFet transistor or an Ion Selective Field Effect Transistor (ISFET) adapted such as its gate is in contact with the medium (3) when the implant is deployed in the medium (3), such as the conductance of the transistor depends on the concentration of chemical species at the gate of the transistor.

11. A sensing system according to any of the claims 1 to 5, wherein the capacitor (8) has a given nominal capacitance, and the implant (1) further comprises an optical reactive material (13), preferably a fluorescence or phosphorescence variable material, arranged in the implant to be in contact with a medium (3) when the implant is deployed in the medium, and wherein an optical property of the optical transducer is variable depending on a physical or chemical condition of the medium, wherein the device of asymmetric conductance (9) is a light emitting semiconductor device, wherein the discharge network (10) comprises a light sensitive conductive device (39), wherein the optical material (13) is arranged such as to transmit, reflect or refract light from the light emitting semiconductor device (9) to the light sensitive conductive device (39).

12. A sensing system according to any of the claims 1 to 5, wherein the capacitor (8) has a given nominal capacitance, and the implant further comprises a transmitting, reflecting, or refractive optical reactive material (13), wherein the optical reactive material is arranged in the implant to be in contact with a medium (3) when the implant is deployed in the medium, and wherein the discharge network (10) is connected in parallel with the device of asymmetric conductance (9), and comprises a light emitting semiconductor device (40), so as to emit light during the capacitor discharge, and a light sensitive conductive device (29) connected in parallel, and wherein the optical reactive material (13) is arranged to transmit, reflect or refract light from the light emitting semiconductor device (40) to the light sensitive conductive device (29).

13. A sensing system according to any of the claims 11 or 12, further comprising optical filters or diffraction grids (27,28) placed on the light emitting semiconductor device (9) or on the light sensitive conductive device (39), so as to select specific light wavelengths or bands of operation.

14. A sensing system according to any of the claims 1 to 5, suitable to be implanted inside an artery or a vein for measuring blood pressure, further comprising a capsule (22) having at least a part made of a flexible material that allows pressure transmission from the capsule exterior to its interior, and wherein the implant circuit (4) is housed within the capsule (22), the implant (1) further comprising two electrodes (5a,5b) passing through the capsule (22) and connected with the implant circuit (4), wherein each electrode (5a,5b) is a flexible structure configured to anchor the implant to the artery or vein, and wherein the capacitor (8) is a capacitive pressure sensor, such as the capacitor and the flexible part of the capsule are arranged relative to each other such as the capacitance of the pressure sensor depends on blood pressure.

15. A sensing system according to any of the claims 1 to 5, wherein the discharge network (10) comprises a current controlling device incorporating a transducer wherein the current control depends on a parameter of the transducer, and wherein optionally the current controlling device is a JFET transistor and a resistive transducer wherein the source of the JFET is connected to one terminal of the resistive transducer and the gate of the JFET transistor is connected to the other terminal of the resistive transducer.

16. A sensing system according to any of the claims 1 to 4, wherein the reading unit (2) is additionally adapted for processing the sensed voltage and/or current, to calculate the capacitance of the implant capacitor, the resistance of the discharging network (10), or the impedance of the medium (3) surrounding the implant (1) when the implant is deployed in the medium, and for processing the calculated value to obtain a measurement of interest.

17. A sensing system according to claim 16, wherein the reading unit (2) is adapted for reading the implant (1) by monitoring the time course of relative changes of the burst current amplitude *iₚₑₐₖ*(*t*) as the implant capacitor (8) charges, for fitting the recorded variations of the time course of relative changes of the burst current amplitude *iₚₑₐₖ*(*t*) to a model by adjusting a characteristic value, and for calculating a desired measurement from the characteristic value.

18. A sensing system according to claim 16, wherein the reading unit (2) is adapted for reading the implant (1) by monitoring the voltage across the reading unit electrodes (6) during the implant capacitor (8) discharge after a burst cessation, for fitting the recorded voltage waveform to a model by adjusting a characteristic value, and for calculating a measurement from the characteristic value.

19. A sensing system according to claim 16, wherein the reading unit (2) is adapted for reading the implant (1) by delivering bursts of different amplitude and monitoring the current unbalances between positive and negative semicycles, for fitting the recorded unbalances to a model by adjusting a characteristic value, and for calculating a measurement from the characteristic value.

20. A sensing system according to any of the preceding claims, wherein the reading unit (2) is an external battery powered hand-held unit, or wherein the reading unit (2) comprises an, or at least a part of it is, implantable sub-unit adapted for reading an implant, and an external sub-unit adapted for presenting information related to the readings of the implantable sub-unit, and wherein the two sub-units are wirelessly communicated.

## Patentansprüche

1. Sensorsystem, das dazu konfiguriert ist, physikalische und/oder chemische Parameter eines lebenden Körpers zu messen, wobei das System Folgendes umfasst:
- mindestens ein Implantat (1), das dazu konfiguriert ist, in ein Medium (3) des lebenden Körpers implantiert zu werden, wobei das Implantat (1) eine elektronische Schaltung (4) und mindestens zwei mit der elektronischen Schaltung (4) verbundene Elektroden (5a, 5b) umfasst, wobei die elektronische Schaltung (4) einen Kondensator (8) und eine Vorrichtung mit asymmetrischer Leitfähigkeit (9) umfasst, die beide in Reihe zwischen den beiden Elektroden (5a, 5b) geschaltet sind, wobei die elektronische Schaltung (4) ferner ein Entladungsnetzwerk (10) umfasst, das parallel zu der Vorrichtung mit asymmetrischer Leitfähigkeit (9) geschaltet ist, wobei das Entladungsnetzwerk (10) dazu konfiguriert ist, den Kondensator (8) unter Umgehung der Vorrichtung mit asymmetrischer Leitfähigkeit (9) zu entladen, wobei das Entladungsnetzwerk (10) mindestens eine elektrische oder elektronische Komponente (11) umfasst, wobei der Kondensator (8) und/oder die Vorrichtung mit asymmetrischer Leitfähigkeit (9) und/oder die elektrische oder elektronische Komponente (11) ein Wandler ist, der einen oder mehrere charakteristische Werte umfasst, die dazu konfiguriert sind, abhängig von einem physischen und/oder chemischen Zustand des Mediums (3) zu variieren, wenn das Implantat (1) in dem Medium (3) eingesetzt ist; und
- eine Leseeinheit (2), die dazu konfiguriert ist, das elektronische Implantat (1) zu lesen, wenn das Implantat (1) im Medium (3) des lebenden Körpers eingesetzt ist, wobei die Leseeinheit (2) zwei oder mehr Leseeinheitselektroden (6), einen Wechselspannungsgenerator (34) zum Generieren einer Wechselspannung über die Leseeinheitselektroden (6) und ein Steuer- und Verarbeitungsmodul (17) umfasst, wobei die Leseeinheitselektroden (6) dazu konfiguriert sind, mit der Haut des lebenden Körpers in Kontakt gebracht zu werden, und wobei die Leseeinheit (2) zu Folgendem konfiguriert ist:
• Emittieren eines Abfragesignals über die Leseeinheitselektroden (6), wobei das Abfragesignal mindestens einen Burst (35) aus Wechselstrom umfasst, der geeignet ist, das Implantat (1) durch Volumenleitung durch das Medium (3) zu erreichen;
• Erkennen, in den Leseeinheitselektroden (6) oder in den mit der elektronischen Schaltung (4) verbundenen Elektroden (5a, 5b), der resultierenden Spannungs- und/oder Stromsignale des Implantats (1), die als Reaktion auf das Abfragesignal generiert werden;
• Verarbeiten, mit dem Steuer- und Verarbeitungsmodul (17), der erkannten Signale, um den einen oder die mehreren charakteristischen Werte des Wandlers abzuleiten; und
• Gewinnen, aus dem einen oder den mehreren abgeleiteten charakteristischen Werten des Wandlers, eines Wertes der physikalischen und/oder chemischen Parameter des zu messenden lebenden Körpers.

2. Sensorsystem nach Anspruch 1, wobei die Leseeinheit (2) dazu angepasst ist, Bursts (35) aus Wechselstrom mit einer Frequenz zwischen 100 kHz und 100 MHz zu emittieren, wobei die Dauer der Bursts (35) zwischen 0,1 µs und 10 ms und die Wiederholungsfrequenz zwischen 0 Hz und 100 kHz liegt.

3. Sensorsystem nach einem der Ansprüche 1 oder 2, wobei das Implantat (1) einen langgestreckten und flexiblen Körper (36) aus einem elektrisch isolierenden Material umfasst, und wobei die elektronische Schaltung (4) innerhalb des Körpers aufgenommen ist, und wobei das Implantat (1) ferner zwei metallische Elektroden (5a, 5b) an den gegenüberliegenden Enden des Körpers umfasst, die elektrisch mit der elektronischen Schaltung verbunden sind, und wobei die Länge des Implantats optional im Bereich von 0,5 cm - 5 cm liegt.

4. Sensorsystem nach einem der Ansprüche 1 bis 3, wobei das Entladungsnetzwerk (10) des Implantats (1) eine Stromsteuerungsvorrichtung zum Steuern des Entladungsstroms des Kondensators (8) umfasst und den Entladungsprozess unabhängig von der Impedanz des Mediums (3') und der Elektroden (5a', 5b') macht.

5. Sensorsystem nach einem der vorhergehenden Ansprüche, wobei die Vorrichtung mit asymmetrischer Leitfähigkeit (9) eine Diode (vorzugsweise eine Schottky-Diode oder eine LED), ein p-n-Übergang eines Transistors oder eine Smart-Diode ist.

6. Sensorsystem nach einem der Ansprüche 1 bis 5, wobei die elektrische oder elektronische Komponente (11) des Entladungsnetzwerks ein Widerstand mit einem gegebenen Nennwert ist und wobei der Kondensator (8) ein Wandler ist, dessen Kapazität abhängig von einem physikalischen und/oder chemischen Zustand des Mediums (3) variabel ist.

7. Sensorsystem nach einem der Ansprüche 1 bis 5, wobei der Kondensator (8) eine gegebene Nennkapazität aufweist und wobei die elektronische Komponente (11) des Entladungsnetzwerks ein resistiver Wandler ist.

8. Sensorsystem nach einem der Ansprüche 1 bis 5, wobei die Kapazität des Kondensators (8) innerhalb des Bereichs von 10 pF bis 10 nF liegt und wobei der Widerstand des Entladungsnetzwerks (10) innerhalb des Bereichs von 1 kΩ bis 10 MΩ liegt.

9. Sensorsystem nach einem der Ansprüche 1 bis 5, das zum Messen von Biopotentialen geeignet ist, wobei der Kondensator (8) eine gegebene Nennkapazität aufweist und wobei das Entladungsnetzwerk (10) einen Transistor (38) umfasst, dessen Gate- oder Basisanschluss so angeordnet ist, dass er mittels einer dritten Elektrode (5c) mit dem Medium (3) in Kontakt steht, sodass die Leitfähigkeit des Transistors von der Spannung an der dritten Elektrode (5c) abhängt.

10. Sensorsystem nach einem der Ansprüche 1 bis 5, das zum Messen chemischer Spezies geeignet ist, wobei der Kondensator (8) eine gegebene Nennkapazität aufweist und wobei das Entladungsnetzwerk (10) einen ChemFet-Transistor oder einen ionenselektiven Feldeffekttransistor (ISFET) umfasst, der so angepasst ist, dass sein Gate mit dem Medium (3) in Kontakt steht, wenn das Implantat in dem Medium (3) eingesetzt ist, sodass die Leitfähigkeit des Transistors von der Konzentration der chemischen Spezies am Gate des Transistors abhängt.

11. Sensorsystem nach einem der Ansprüche 1 bis 5, wobei der Kondensator (8) eine gegebene Nennkapazität aufweist und das Implantat (1) ferner ein optisch reaktives Material (13) umfasst, vorzugsweise ein Material mit variabler Fluoreszenz oder Phosphoreszenz, das im Implantat so angeordnet ist, dass es mit einem Medium (3) in Kontakt steht, wenn das Implantat in dem Medium eingesetzt ist, und wobei eine optische Eigenschaft des optischen Wandlers abhängig von einem physischen oder chemischen Zustand des Mediums variabel ist, wobei die Vorrichtung mit asymmetrischer Leitfähigkeit (9) eine lichtemittierende Halbleitervorrichtung ist, wobei das Entladungsnetzwerk (10) eine lichtempfindliche leitfähige Vorrichtung (39) umfasst, wobei das optische Material (13) so angeordnet ist, dass es Licht von der lichtemittierenden Halbleitervorrichtung (9) zur lichtempfindlichen leitfähigen Vorrichtung (39) durchlässt, reflektiert oder bricht.

12. Sensorsystem nach einem der Ansprüche 1 bis 5, wobei der Kondensator (8) eine gegebene Nennkapazität aufweist und das Implantat ferner ein durchlässiges, reflektierendes oder brechendes optisch reaktives Material (13) umfasst, wobei das optisch reaktive Material in dem Implantat so angeordnet ist, dass es mit einem Medium (3) in Kontakt steht, wenn das Implantat in dem Medium eingesetzt ist, und wobei das Entladungsnetzwerk (10) parallel zu der Vorrichtung mit asymmetrischer Leitfähigkeit (9) geschaltet ist, und eine lichtemittierende Halbleitervorrichtung (40), um während der Kondensatorentladung Licht zu emittieren, und eine parallel geschaltete lichtempfindliche leitfähige Vorrichtung (29) umfasst, und wobei das optisch reaktive Material (13) so angeordnet ist, dass es Licht von der lichtemittierenden Halbleitervorrichtung (40) zur lichtempfindlichen leitfähigen Vorrichtung (29) durchlässt, reflektiert oder bricht.

13. Sensorsystem nach einem der Ansprüche 11 oder 12, ferner umfassend optische Filter oder Beugungsgitter (27, 28), die auf der lichtemittierenden Halbleitervorrichtung (9) oder auf der lichtempfindlichen leitfähigen Vorrichtung (39) platziert sind, um spezifische Lichtwellenlängen oder Betriebsbänder auszuwählen.

14. Sensorsystem nach einem der Ansprüche 1 bis 5, das zur Implantation innerhalb einer Arterie oder Vene zur Blutdruckmessung geeignet ist, ferner umfassend eine Kapsel (22), die mindestens einen Teil aus einem flexiblen Material aufweist, das eine Druckübertragung von der Außenseite der Kapsel zum Inneren derselben ermöglicht, und wobei die Implantatschaltung (4) innerhalb der Kapsel (22) aufgenommen ist, wobei das Implantat (1) ferner zwei Elektroden (5a, 5b) umfasst, die durch die Kapsel (22) verlaufen und mit der Implantatschaltung (4) verbunden sind, wobei jede Elektrode (5a, 5b) eine flexible Struktur ist, die dazu konfiguriert ist, das Implantat in der Arterie oder Vene zu verankern, und wobei der Kondensator (8) ein kapazitiver Drucksensor ist, sodass der Kondensator und der flexible Teil der Kapsel derart relativ zueinander angeordnet sind, dass die Kapazität des Drucksensors vom Blutdruck abhängt.

15. Sensorsystem nach einem der Ansprüche 1 bis 5, wobei das Entladungsnetzwerk (10) eine Stromsteuerungsvorrichtung umfasst, die einen Wandler beinhaltet, wobei die Stromsteuerung von einem Parameter des Wandlers abhängt, und wobei die Stromsteuerungsvorrichtung optional ein JFET-Transistor und ein resistiver Wandler ist, wobei die Quelle des JFET mit einem Anschluss des resistiven Wandlers verbunden ist und das Gate des JFET-Transistors mit dem anderen Anschluss des resistiven Wandlers verbunden ist.

16. Sensorsystem nach einem der Ansprüche 1 bis 4, wobei die Leseeinheit (2) zusätzlich dazu angepasst ist, die erfasste Spannung und/oder den erfassten Strom zu verarbeiten, um die Kapazität des Implantatkondensators, den Widerstand des Entladungsnetzwerks (10) oder die Impedanz des das Implantat (1) umgebenden Mediums (3) zu berechnen, wenn das Implantat in dem Medium eingesetzt ist, und den berechneten Wert zu verarbeiten, um eine Messung von Interesse zu gewinnen.

17. Sensorsystem nach Anspruch 16, wobei die Leseeinheit (2) dazu angepasst ist, das Implantat (1) zu lesen, indem der zeitliche Verlauf der relativen Änderungen der Burststromamplitude *iₚₑₐₖ*(*t*) während des Aufladens des Implantatkondensators (8) überwacht wird, die aufgezeichneten Variationen des zeitlichen Verlaufs der relativen Änderungen der Burststromamplitude *iₚₑₐₖ*(*t*) durch Einstellen eines charakteristischen Werts an ein Modell anzupassen und aus dem charakteristischen Wert eine gewünschte Messung zu berechnen.

18. Sensorsystem nach Anspruch 16, wobei die Leseeinheit (2) dazu angepasst ist, das Implantat (1) zu lesen, indem die Spannung über den Leseeinheitselektroden (6) während der Entladung des Implantatkondensators (8) nach Beendigung eines Bursts überwacht wird, die aufgezeichnete Spannungswellenform durch Einstellen eines charakteristischen Werts an ein Modell anzupassen und aus dem charakteristischen Wert eine Messung zu berechnen.

19. Sensorsystem nach Anspruch 16, wobei die Leseeinheit (2) dazu angepasst ist, das Implantat (1) zu lesen, indem Bursts unterschiedlicher Amplitude abgegeben und die Stromungleichgewichte zwischen positiven und negativen Halbzyklen überwacht werden, die aufgezeichneten Ungleichgewichte durch Einstellen eines charakteristischen Werts an ein Modell anzupassen und aus dem charakteristischen Wert eine Messung zu berechnen.

20. Sensorsystem nach einem der vorhergehenden Ansprüche, wobei die Leseeinheit (2) eine externe, batteriebetriebene tragbare Einheit ist oder wobei die Leseeinheit (2) eine implantierbare Untereinheit, die zum Auslesen eines Implantats angepasst ist, oder mindestens einen Teil davon umfasst und eine externe Untereinheit, die zum Präsentieren von Informationen im Zusammenhang mit den Auslesewerten der implantierbaren Untereinheit angepasst ist, und wobei die beiden Untereinheiten drahtlos miteinander kommunizieren.

## Revendications

1. Système de détection configuré pour mesurer des paramètres physiques et/ou chimiques d'un organisme vivant, le système comprenant :
- au moins un implant (1), configuré pour être implanté dans un milieu (3) de l'organisme vivant, ledit implant (1) comprenant un circuit électronique (4) et au moins deux électrodes (5a, 5b) connectées au circuit électronique (4), dans lequel le circuit électronique (4) comprend un condensateur (8) et un dispositif à conductance asymétrique (9), tous deux connectés en série entre les deux électrodes (5a, 5b), le circuit électronique (4) comprenant en outre un réseau de décharge (10) connecté en parallèle avec le dispositif à conductance asymétrique (9), le réseau de décharge (10) étant configuré pour décharger le condensateur (8) en contournant le dispositif à conductance asymétrique (9), dans lequel le réseau de décharge (10) comprend au moins un composant électrique ou électronique (11), dans lequel le condensateur (8) et/ou le dispositif à conductance asymétrique (9) et/ou le composant électrique ou électronique (11) constitue un transducteur comprenant une ou plusieurs valeurs caractéristiques configurées pour varier en fonction d'une condition physique et/ou chimique du milieu (3) lorsque l'implant (1) est déployé dans le milieu (3) ; et
- une unité de lecture (2) configurée pour lire l'implant électronique (1) lorsque l'implant (1) est déployé dans le milieu (3) de l'organisme vivant, dans lequel l'unité de lecture (2) comprend deux ou plusieurs électrodes d'unité de lecture (6), un générateur de tension alternative (34) pour générer une tension alternative aux bornes des électrodes d'unité de lecture (6), et un module de commande et de traitement (17), dans lequel les électrodes d'unité de lecture (6) sont configurées pour être placées en contact avec la peau de l'organisme vivant, et dans lequel l'unité de lecture (2) est configurée pour :
• émettre un signal d'interrogation par l'intermédiaire des électrodes d'unité de lecture (6), ledit signal d'interrogation comprenant au moins une salve (35) d'un courant alternatif apte à atteindre l'implant (1) par conduction volumique à travers le milieu (3) ;
• détecter, au niveau des électrodes d'unité de lecture (6) ou des électrodes (5a, 5b) connectées au circuit électronique (4), des signaux de tension et/ou de courant résultants provenant de l'implant (1), générés en réponse au signal d'interrogation ;
• traiter, au moyen du module de commande et de traitement (17), lesdits signaux détectés afin de déduire la ou les valeurs caractéristiques du transducteur ; et
• obtenir, à partir de la ou des valeurs caractéristiques déduites du transducteur, une valeur des paramètres physiques et/ou chimiques de l'organisme vivant à mesurer.

2. Système de détection selon la revendication 1, dans lequel l'unité de lecture (2) est adaptée pour émettre des salves (35) de courant alternatif à une fréquence comprise entre 100 kHz et 100 MHz, avec une durée des salves (35) comprise entre 0,1 µs et 10 ms, et une fréquence de répétition comprise entre 0 Hz et 100 kHz.

3. Système de détection selon l'une quelconque des revendications 1 ou 2, dans lequel l'implant (1) comprend un corps allongé et flexible (36) constitué d'un matériau électriquement isolant, et dans lequel le circuit électronique (4) est logé à l'intérieur du corps, et dans lequel l'implant (1) comprend en outre deux électrodes métalliques (5a, 5b) aux extrémités opposées du corps, qui sont électriquement connectées au circuit électronique, et dans lequel, facultativement, la longueur de l'implant est comprise dans la plage de 0,5 cm à 5 cm.

4. Système de détection selon l'une quelconque des revendications 1 à 3, dans lequel le réseau de décharge (10) de l'implant (1) comprend un dispositif de commande de courant destiné à commander le courant de décharge du condensateur (8) et rend le processus de décharge indépendant de l'impédance du milieu (3') et des électrodes (5a', 5b').

5. Système de détection selon l'une quelconque des revendications précédentes, dans lequel le dispositif à conductance asymétrique (9) est une diode, de préférence une diode Schottky ou une DEL, une jonction p-n d'un transistor ou une diode intelligente.

6. Système de détection selon l'une quelconque des revendications 1 à 5, dans lequel le composant électrique ou électronique (11) du réseau de décharge est une résistance ayant une valeur nominale donnée, et dans lequel le condensateur (8) est un transducteur dont la capacité est variable en fonction d'une condition physique et/ou chimique du milieu (3).

7. Système de détection selon l'une quelconque des revendications 1 à 5, dans lequel le condensateur (8) présente une capacité nominale donnée, et dans lequel le composant électronique (11) du réseau de décharge est un transducteur résistif.

8. Système de détection selon l'une quelconque des revendications 1 à 5, dans lequel la capacité du condensateur (8) est comprise dans la plage de 10 pF à 10 nF, et dans lequel la résistance du réseau de décharge (10) est comprise dans la plage de 1 kΩ à 10 MΩ.

9. Système de détection selon l'une quelconque des revendications 1 à 5, apte à mesurer des biopotentiels, dans lequel le condensateur (8) présente une capacité nominale donnée, et dans lequel le réseau de décharge (10) comprend un transistor (38), dont la borne de grille ou de base est agencée pour être en contact avec le milieu (3) au moyen d'une troisième électrode (5c), de sorte que la conductance du transistor dépend de la tension au niveau de la troisième électrode (5c).

10. Système de détection selon l'une quelconque des revendications 1 à 5, apte à mesurer des espèces chimiques, dans lequel le condensateur (8) présente une capacité nominale donnée, et dans lequel le réseau de décharge (10) comprend un transistor ChemFet ou un transistor à effet de champ sélectif aux ions (ISFET), adapté de sorte que sa grille soit en contact avec le milieu (3) lorsque l'implant est déployé dans le milieu (3), de sorte que la conductance du transistor dépend de la concentration en espèces chimiques au niveau de la grille du transistor.

11. Système de détection selon l'une quelconque des revendications 1 à 5, dans lequel le condensateur (8) présente une capacité nominale donnée, et l'implant (1) comprend en outre un matériau optiquement réactif (13), de préférence un matériau à fluorescence ou à phosphorescence variable, agencé dans l'implant de manière à être en contact avec un milieu (3) lorsque l'implant est déployé dans le milieu, et dans lequel une propriété optique du transducteur optique est variable en fonction d'une condition physique ou chimique du milieu, dans lequel le dispositif à conductance asymétrique (9) est un dispositif semi-conducteur émettant de la lumière, dans lequel le réseau de décharge (10) comprend un dispositif conducteur photosensible (39), dans lequel le matériau optique (13) est agencé de manière à transmettre, réfléchir ou réfracter la lumière provenant du dispositif semi-conducteur émettant de la lumière (9) vers le dispositif conducteur photosensible (39).

12. Système de détection selon l'une quelconque des revendications 1 à 5, dans lequel le condensateur (8) présente une capacité nominale donnée, et l'implant comprend en outre un matériau optiquement réactif transmettant, réfléchissant ou réfractant (13), dans lequel le matériau optiquement réactif est agencé dans l'implant de manière à être en contact avec un milieu (3) lorsque l'implant est déployé dans le milieu, et dans lequel le réseau de décharge (10) est connecté en parallèle avec le dispositif à conductance asymétrique (9) et comprend un dispositif semi-conducteur émettant de la lumière (40), de manière à émettre de la lumière pendant la décharge du condensateur, ainsi qu'un dispositif conducteur photosensible (29) connecté en parallèle, et dans lequel le matériau optiquement réactif (13) est agencé pour transmettre, réfléchir ou réfracter la lumière provenant du dispositif semi-conducteur émettant de la lumière (40) vers le dispositif conducteur photosensible (29).

13. Système de détection selon l'une quelconque des revendications 11 ou 12, comprenant en outre des filtres optiques ou des réseaux de diffraction (27, 28) placés sur le dispositif semi-conducteur émettant de la lumière (9) ou sur le dispositif conducteur photosensible (39), de manière à sélectionner des longueurs d'onde lumineuses ou des bandes de fonctionnement spécifiques.

14. Système de détection selon l'une quelconque des revendications 1 à 5, apte à être implanté à l'intérieur d'une artère ou d'une veine pour mesurer la pression sanguine, comprenant en outre une capsule (22) ayant au moins une partie constituée d'un matériau flexible permettant la transmission de pression depuis l'extérieur de la capsule vers son intérieur, et dans lequel le circuit de l'implant (4) est logé à l'intérieur de la capsule (22), l'implant (1) comprenant en outre deux électrodes (5a, 5b) traversant la capsule (22) et connectées au circuit de l'implant (4), dans lequel chaque électrode (5a, 5b) est une structure flexible configurée pour ancrer l'implant à l'artère ou à la veine, et dans lequel le condensateur (8) est un capteur de pression capacitif, de sorte que le condensateur et la partie flexible de la capsule sont agencés l'un par rapport à l'autre de sorte que la capacité du capteur de pression dépende de la pression sanguine.

15. Système de détection selon l'une quelconque des revendications 1 à 5, dans lequel le réseau de décharge (10) comprend un dispositif de commande de courant incorporant un transducteur, dans lequel la commande de courant dépend d'un paramètre du transducteur, et dans lequel, facultativement, le dispositif de commande de courant est un transistor JFET et un transducteur résistif, dans lequel la source du JFET est connectée à une borne du transducteur résistif et la grille du transistor JFET est connectée à l'autre borne du transducteur résistif.

16. Système de détection selon l'une quelconque des revendications 1 à 4, dans lequel l'unité de lecture (2) est en outre adaptée pour traiter la tension et/ou le courant détectés, afin de calculer la capacité du condensateur de l'implant, la résistance du réseau de décharge (10), ou l'impédance du milieu (3) entourant l'implant (1) lorsque l'implant est déployé dans le milieu, et pour traiter la valeur calculée afin d'obtenir une mesure d'intérêt.

17. Système de détection selon la revendication 16, dans lequel l'unité de lecture (2) est adaptée pour lire l'implant (1) en surveillant l'évolution temporelle des variations relatives de l'amplitude du courant de salve *iₚₑₐₖ*(*t*) à mesure que le condensateur de l'implant (8) se charge, pour ajuster les variations enregistrées de l'évolution temporelle des variations relatives de l'amplitude du courant de salve *iₚₑₐₖ*(*t*) à un modèle en ajustant une valeur caractéristique, et pour calculer une mesure souhaitée à partir de la valeur caractéristique.

18. Système de détection selon la revendication 16, dans lequel l'unité de lecture (2) est adaptée pour lire l'implant (1) en surveillant la tension aux bornes des électrodes de l'unité de lecture (6) pendant la décharge du condensateur de l'implant (8) après l'arrêt d'une salve, pour ajuster la forme d'onde de tension enregistrée à un modèle en ajustant une valeur caractéristique, et pour calculer une mesure à partir de la valeur caractéristique.

19. Système de détection selon la revendication 16, dans lequel l'unité de lecture (2) est adaptée pour lire l'implant (1) en délivrant des salves d'amplitudes différentes et en surveillant les déséquilibres de courant entre les demi-cycles positifs et négatifs, pour ajuster les déséquilibres enregistrés à un modèle en ajustant une valeur caractéristique, et pour calculer une mesure à partir de la valeur caractéristique.

20. Système de détection selon l'une quelconque des revendications précédentes, dans lequel l'unité de lecture (2) est une unité portative externe alimentée par batterie, ou dans lequel l'unité de lecture (2) comprend une sous-unité implantable adaptée pour lire un implant, ou dont au moins une partie constitue une telle sous-unité implantable, ainsi qu'une sous-unité externe adaptée pour présenter des informations relatives aux lectures de la sous-unité implantable, et dans lequel les deux sous-unités communiquent sans fil.
